# EUROPEAN PATENT APPLICATION

(11) **EP 1 065 275 A1**
(43) Date of publication of application: **03.01.2001**
(21) Application number: 00114196.9
(22) Date of filing: 22.05.1992
(51) Int. Cl.: C12N 15/32, C07K 14/325, A01N 63/00

(54) **Novel bacillus thuringiensis isolates active against hymenopteran pests and genes encoding hymenopteran-active toxins**

(30) Priority: 22.05.1991 US 703997; 25.11.1991 US 797645
(62) Divisional of application: 92913802.2
(71) Applicant: MYCOGEN CORPORATION, San Diego, California 92121 (US)
(72) Inventor: Payne, Jewel M., San Diego, CA 92126 (US); Kennedy, M. Keith, Racine, WI 53402 (US); Randall, John B., Racine, WI 53406 (US); Meier, Henry, Racine, WI 53406 (US); Uick, Hiedi J., Racine, WI 53406 (US)
(74) Representative: Perry, Robert Edward

(57) **Abstract**

Novel *Bacillus thuringiensis* isolates with hymenopteran activity are described. Also described are toxins having the advantageous hymenopteran activity.

This invention further concerns genes or gene fragments which have been cloned from the novel *Bacillus thuringiensis* isolates which have formicidal activity. These genes or gene fragments can be used to transform suitable hosts for controlling ants.

## Description

### Cross-Reference to a Related Application

This is a continuation-in-part of co-pending application Serial No. 07/703,977, filed on May 22, 1991. This is also a continuation-in-part of application Serial No. 07/797,645, filed on November 25, 1991

### Background of the Invention

The development of biological control agents as alternatives to chemical insecticides for the control of important pest species is a subject of increasing interest. Concerns for the environment and exposure of man to harmful substances in air, food and water have stimulated legislation and restrictions regarding the use of chemical pesticides, particularly for pests found in the urban environment. Control of insect pests in urban areas is highly desirable but exposure to chemical pesticides in the household and from lawns and gardens is of great concern to the public. If given a choice, most people would use a non-toxic biological control rather than a toxic chemical to control insects in the urban environment. The problem is that very few biological alternatives to chemical insecticides are available for purchase and use by the public.

*Bacillus thuringiensis (B.t.)* produces an insect toxin designated as δ-endotoxin. It is synthesized by the *B.t.* sporulating cell. The toxin, upon being ingested in its crystalline form by susceptible insects, is transformed into biologically active moieties by the insect gut juice proteases. The primary target is insect cells of the gut epithelium which are rapidly destroyed.

The reported activity spectrum of *B.t.* covers insect species within the order Lepidoptera, many of which are major pests in agriculture and forestry. The activity spectrum also includes the insect order Diptera, which includes mosquitos and black flies. See Couch, T.L. (1980) "Mosquito Pathogenicity of *Bacillus thuringiensis* var. *israelensis," Developments in Industrial Microbiology* 22:61-76; Beegle, C.C., (1978) "Use of Entomogenous Bacteria in Agroecosystems," *Developments in Industrial Microbiology* 20:97-104. Krieg. *et al.* (1983) *Z. ang. Ent.* 96:500.508, describe a *B.t.* isolate named *Bacillus thuringiensis* var. *tenebrionis,* which is reportedly active against two beetles in the order Coleoptera. These are the Colorado potato beetle, *Leptinotarsa decemlineata*, and *Agelastica alni.* In European Patent Application No. 0 202 739 there is disclosed a novel *B.t.* isolate active against Coleoptera. It is known as *B. thuringiensis* var. san diego (*B.t.s.d.*). U.S. Patent No. 4,966,765 discloses the coleopteran-active *Bacillus thuringiensis* isolate *B.t.* PS86B1.

Ants comprise a large group of insects (family Formicidae) from the taxonomic order, Hymenoptera. They are among the most common house pests. In many situations, ants are a nuisance pest. Foraging ants create problems with hygiene in hospitals and the food industry. Ants also create problems in agriculture Damage can be caused by direct feeding on plants. Harvester and fire ants are commonly associated with this type of damage (Holldobler, B., EO. Wilson [1990] *The Ants*, Belkap Press, Cambridge, Mass. 732 pp.) Some ants cause indirect damage by nurturing and protecting sap feeding insects such as mealybugs and aphids. Ants, particularly in the genus *Solenopsis* are capable of producing extremely painful stings to humans. It has been estimated that approximately 10,000 stings occur each year (Habermehl, G.G. [1981] *Venomous Animals and Their Toxins*, Springer-Verlag, NY, 195 pp.). The pharaoh ant (*Monomorium pharaonis*) is primarily an urban pest. However, this species can also be an agricultural pest and damage to corn has been noted (Ebeling, W. [1978] *Urban Entomology,* UC Press, Berkeley, Calif, 695 pp.).

Carpenter ants, *Camponotus* spp., are distributed throughout North America. Some of the more common and/or studied species include *C. modoc* in the Pacific northwest, *C. clarithorax* in southern California, and the black, red, and Florida carpenter ants, *C. pennsylvanicus, C. noveboracensis* and *C. abdominalis,* respectively, in the east (Ebeling, W. [1978] *Urban Entomology,* Univ. Calif: Berkeley p. 209-213). Public concern over carpenter ants has been increasing due to the greater probability of structural infestations as suburban developments extend into the forest habitats of the ants.

Pestiferous species of carpenter ants may be considered nuisance pests because of their foraging activity inside homes. More significant damage occurs when carpenter ants extend their nests into sound wood. Nesting sites may be located in live and dead trees, sometimes resulting in damage to shade trees. Nests may also be established in walls and support beams of structures, or in voids within doors, walls, and furniture. Preference for moist or decaying wood has been reported, but nesting sites are not restricted to such areas. Carpenter ant populations develop relatively slowly with colonies of 300-2,000 workers being produced over a 2-year or longer period for various species. The presence of reproductives follows this slow development since their production has been reported only from well established colonies (Hansen, L.D., R.D. Akre [1985] Biology of carpenter ants in Washington state (Hymenoptera: Formicidae: *Camponotus*). Melanderia 43. 62 p.; Pricer, J.L. [1908] Biol. Bull. 14:177-218). Despite the slow colony growth, large colonies with satellite colonies have been found. Worker movement occurs between the main colony and the satellites, which serve as areas for further brood development and colony expansion (Hansen and Akre [1985], *supra*).

Current methods for controlling structural infestations of carpenter ants include sanitation of potential and current nest sites, minimizing access to structures (eg. preventing the contact of tree branches with a structure), and the application of insecticides to repel (perimeter spray barriers) and/or eliminate carpenter ants. The use of boric add dust in dry, wall voids is reported to be effective for up to 20 years (Hansen and Akre, *supra*).

Recommendations for the chemical control of established structural infestations in the home are often accompanied with warnings of possible hazards to the applicator as well as children and pets. Alternative control methods such as effective biological control agents have not been found (Akre, RD., L.D. Hansen, A.L. Antonelli [1989] Ext. Bull. Washington State Univ. Coop. Ext. Serv. 1989 rev. no. EB 0818, 6 pp.).

A need clearly exists for a safe, effective biological control agent for carpenter ants.

Pharaoh ants, *Monomorium pharaonis*, have been described as "... the most persistent and difficult of all our house-infesting ants to control or eradicate" (Smith, M.R. [1965] USDAARS Tech. Bull. No. 1324 105 pp.). It is a tropical species which has extended its range to more temperate regions by establishing colonies in heated buildings. Pharaoh ants frequently infests buildings where food is prepared, and have been found to carry pathogenic organisms (Beatson, S.H. [1972] Lancet 1:425-427).

The difficulty in controlling pharaoh ants may be attributed to their inaccessible nesting sites, rapid population growth, and dispersion of colonies. Their small size allows establishment of colonies in any suitable location, including unusual places such as between books and in stored clothing. With multiple queen colonies, and the warm (30°C), humid (63-80% RH) conditions that favor pharaoh ants, large colonies can develop rapidly. Portions of these large colonies may disperse to form new colonies at any time, probably in response to overcrowding and unfavorable microenvironmental conditions. Unlike other ant species, pharaoh ants do not exhibit intercolony aggression. This permits the adoption of ants from other colonies and may further enhance the establishment of new colonies and reinfestations. Pharaoh ants also forage for food more than 35 m from the nest without distinct trail following, and thus make nests difficult to find and eradicate.

Control methods for pharaoh ants emphasize the use of insect growth regulators (IGR) or toxicants incorporated into baits. Properly implemented bait programs are effective, however it may take over a month to achieve control. Insecticide applications, while fast acting, usually do not eliminate colonies, and may be unacceptable in certain areas where toxic residues are a concern. In addition, insecticide applications are generally not compatible with bait programs.

A need exists for safe and effective biological control agents for pharaoh ants.

### Brief Summary of the Invention

The subject invention concerns novel *Bacillus thuringiensis* (*B.t.*) isolates and genes therefrom which encode novel hymenopteran-active proteins. The novel *B.t,* isolates, known herein as *Bacillus thuringiensis* PS140E2 (*B.t.* PS140E2), *Bacillus thuringiensis* PS86Q3 (*B.t.* PS86Q3) and *Bacillus thuringiensis* PS211B2 (*B.t,* PS211B2) have been shown to be active against, for example, the pharaoh ant (*Monomorium pharaonis*). Toxins of the subject invention control, for example, fire ants, carpenter ants, argentine ants, and pharaoh ants.

The subject invention also includes mutants of the above isolates which have substantially the same pesticidal properties as the parent isolate. Procedures for making mutants are well known in the microbiological art. Ultraviolet light and nitrosoguanidine are used extensively toward this end.

The subject invention also concerns novel toxins active against ants. A further aspect of the invention concerns genes coding for these formicidal toxins. The subject invention provides the person skilled in this art with a vast array of formicidal toxins, methods for using these toxins, and genes that code for the toxins. The genes or gene fragments of the invention encode *Bacillus thuringiensis* δ-endotoxins which have formicidal activity. The genes or gene fragments can be transferred to suitable hosts via a recombinant DNA vector.

One aspect of the invention is the discovery of a generalized chemical formula common to a wide range of formicidal toxins. This formula can be used by those skilled in this art to obtain and identify a wide variety of toxins having the desired formicidal activity. The subject invention concerns other teachings which enable the skilled practitioner to identify and isolate ant-active toxins and the genes which code therefor. For example, characteristic features of ant-active toxin crystals are disclosed herein. Furthermore, characteristic levels of amino acid homology can be used to characterize the toxins of the subject invention. Yet another characterizing feature pertains to immunoreactivity with certain antibodies. Also, nucleotide probes specific for genes encoding toxins with formicidal activity are described. Thus, the identification of toxins of the subject invention can be accomplished by sequence-specific motifs, overall sequence similarity, immunoreactivity, and ability to hybridize with specific probes.

In addition to the teachings of the subject invention which broadly define *B.t.* toxins with advantageous formicidal activity, a further aspect of the subject invention is the provision of specific formicidal toxins and the nucleotide sequences which code for these toxins. One such toxin is the gene expression product of isolate PS86Q3.

### Brief Description of the Drawings

**Figure 1** is a photograph of a standard SDS polyacrylamide gel of *B.t.* PS140E2, and *B.t.* PS86Q3.
**Figure 2** is a photograph of a standard SDS polyacrylamide gel showing alkali-soluble proteins of *B.t.* PS211B2 compared to a protein standard.
**Figures 3-5** are transmission electron micrographs of ultrathin sections of the ant-active *B.t.* strains (Figure 3 is *B.t.* PS14E2; Figure 4 is *B.t.* PS86Q3; and Figure 5 is *B.t.* PS211B2). Cells were embedded in an epoxy resin and stained with uranyl acetate and lead citrate.

### Brief Description of the Sequences

**SEQ ID NO.1** is the nucleotide sequence of gene 17a.
**SEQ ID NO. 2** is the amino acid sequence of protein 17a.
**SEQ ID NO. 3** is the nucleotide sequence of gene 17b.
**SEQ ID NO. 4** is the amino acid sequence of protein 17b.
**SEQ ID NO. 5** is the nucleotide sequence of gene 33F2.
**SEQ ID NO. 6** is the amino acid sequence of protein 33F2.
**SEQ ID NO. 7** is the nucleotide sequence of gene 86Q3(a).
**SEQ ID NO. 8** is the amino acid sequence of protein 86Q3(a).
**SEQ ID NO. 9** is the nucleotide sequence of gene 63B.
**SEQ ID NO. 10** is the amino acid sequence of protein 63B.
**SEQ ID NO. 11** is the amino add sequence of a probe which can be used according to the subject invention.
**SEQ ID NO. 12** is DNA coding for the amino acid sequence of SEQ ID NO. 11.
**SEQ ID NO. 13** is DNA coding for the amino acid sequence of SEQ ID NO. 11.
**SEQ ID NO. 14** is the amino acid sequence of a probe which can be used according to the subject invention.
**SEQ ID NO. 15** is DNA coding for the amino acid sequence of SEQ ID NO. 14.
**SEQ ID NO. 16** is DNA coding for the amino acid sequence of SEQ ID NO. 14.
**SEQ ID NO. 17** is the N-terminal amino acid sequence of 17a.
**SEQ ID NO. 18** is the N-terminal amino acid sequence of 17b.
**SEQ ID NO. 19** is the N-terminal amino acid sequence of 86Q3(a).
**SEQ ID NO. 20** is the N-terminal amino acid sequence of 63B.
**SEQ ID NO. 21** is the N-terminal amino acid sequence of 33F2.
**SEQ ID NO. 22** is an internal amino acid sequence for 63B.
**SEQ ID NO. 23** is a synthetic oligonucleotide derived from 17.
**SEQ ID NO. 24** is the forward oligonucleotide primer from 63B.
**SEQ ID NO. 25** is the reverse oligonucleotide primer from 63B.
**SEQ ID NO. 26** is oligonucleotide probe 33F2A.
**SEQ ID NO. 27** is oligonucleotide probe 33F2B.
**SEQ ID NO. 28** is a reverse primer used according to the subject invention.
**SEQ ID NO. 29** is an oligonucleotide derived from the N-terminal amino acid sequence of 86Q3(a) (SEQ ID NO. 19).
**SEQ ID NO. 30** is the amino acid sequence coded for by an oligonucleotide used according to the subject invention (SEQ ID NO. 31).
**SEQ ID NO. 31** is an oligonucleotide which codes for the amino acid sequence of SEQ ID NO. 30.
**SEQ ID NO. 32** is the amino acid sequence coded for by the oligonucleotide of SEQ ID NO. 33.
**SEQ ID NO. 33** is a DNA sequence coding for the peptide of SEQ ID NO. 32.
**SEQ ID NO. 34** is the reverse complement primer to SEQ ID NO. 38, used according to the subject invention.
**SEQ ID NO. 35** is a forward primer according to the subject invention.
**SEQ ID NO. 36** is an amino acid sequence according to the subject invention.
**SEQ ID NO. 37** is a reverse primer according to the subject invention.
**SEQ ID NO. 38** is the nematode (NEMI) variant of region 5 of Höfte and Whiteley.

### Detailed Disclosure of the Invention

One aspect of the subject invention is the discovery of *Bacillus thuringiensis* isolates having activity against ants. The novel *Bacillus thuringiensis* isolates of the subject invention have the following characteristics in their biologically pure form:

### Characteristics of B.t. PS140E2

Colony morphology--large colony, dull surface, typical *B.t.*
Vegetative cell morphology--typical *B.t.*
Culture methods--typical for *B.t.*
Inclusions--an elliptical coated inclusion outside the exosporium, and a long inclusion inside the exosporium
Approximate molecular weight of alkali/SDS-soluble polypeptides (kDa)--78, 70, 35
Serotype-6, entomocidus.

### Characteristics of B.t. PS86Q3

Colony morphology--large colony, dull surface, typica*l B.t.*
Vegetative cell morphology--typical *B.t.*
Culture methods--typical for *B.t.*
Inclusions--long amorphic inclusion and a small inclusion, both of which remain with the spore after lysis
Approximate molecular weight of alkali/SDS-soluble polypeptides (kDa)--155, 135, 98, 62,58
Serotype--new serotype (not H-1 through H-27).

### Characteristics of B.t. PS211B2

Colony morphology--large colony, dull surface, typical *B.t.*
Vegetative cell morphology--typical *B.t.*
Culture methods--typical for *B.t.*
Inclusions--large round amorphic inclusion with coat, and elliptical inclusion
Approximate molecular weight of alkali/SDS-soluble polypeptides (kDa)--175, 130, 100, 83, 69, 43, 40, 36, 35, 34 and 27
Serotype-6, entomocidus.

A comparison of the characteristics of *B. thuringiensis* PS140E2 (*B.t.* PS140E2), *B. thuringiensis* PS86Q3 (*B.t.* PS86Q3), *B. thuringiensis* PS211B2 (*B.t.* PS211B2), *B. thuringiensis* var. san diego (*B.t.s.d.*), and *B. thuringiensis* var. *kurstaki* (HD-1) is shown in Table 1.

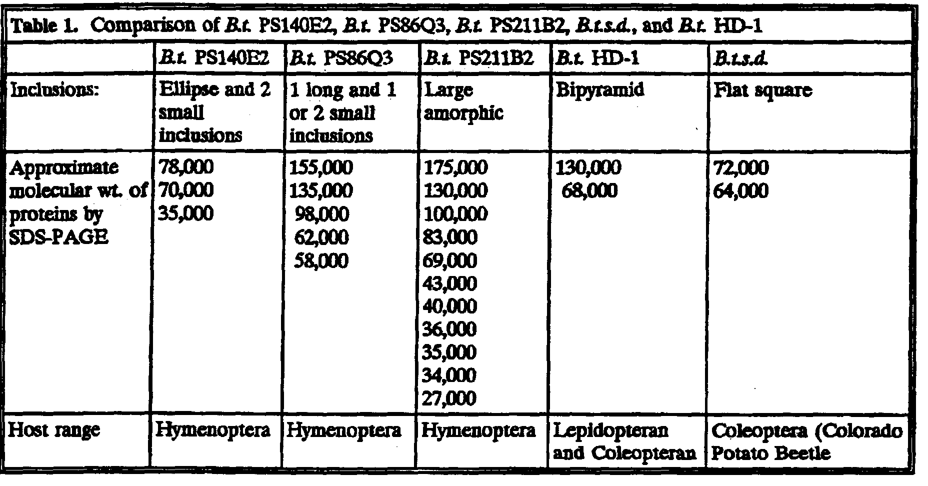

In addition to the ant-active *B.t*. isolates described herein, the subject invention concerns a vast array of *B.t.* δ-endotoxins having formicidal activity. In addition to having formicidal activity, the toxins of the subject invention will have one or more of the following characteristics:
1. An amino add sequence according to the genetic formula disclosed herein.
2. A high degree of amino acid homology with specific toxins disclosed herein.
3. A DNA sequence encoding the toxin wherein said sequence hybridizes with probes or genes disclosed herein.
4. A nucleotide sequence which can be amplified using primers disclosed herein.
5. A crystal toxin presentation as described herein.
6. Immunoreactivity to an antibody raised to a toxin disclosed herein.

One aspect of the subject invention concerns the discovery of a generic chemical formula (hereinafter referred to as the Generic Formula) which can be used to identify toxins having activity against ants. This formula describes toxin proteins having molecular weights in excess of 130,000 kDa. The Generic Formula below covers those amino acids in the N-terminal region extending two amino acids past the invariant proline residue encountered at amino acid number 695 in the sequence of 86Q3(a). The organization of the toxins within this class is delineated by the following generic sequence motif that is the ultimate determinant of structure and function.

1 MOXLUEBYPx BXYUBLXxxx xxxxXXXXXX XXXXXBXXxX EXXXKXXXKX
XxxxxxXJXX XXBXXXXXXX XXLXXXXXXX XXLZBLZBxB PXXXXXXXXX

101 XXBBXXBXXX XXXXXXXXKX xxLBXXBXXX BXXBBXXXBX XXXXXXXUXX
BXZLUXXXXX XXXOBXXXX* XXXXxxxxxx xxxxxxxxxX XX*xxxxxxx

201 xxxxxXXUZX XOXXLXXBxx xxxxxxxXXE XXXXxxxXXL PXYOXBOXXH
LBLXJXXLxx xxxxxXKXXB XXJXxBXXXK XXLXXXLXXX XLOBXXXBXX

301 XLXXXxXXXJ xXZXXXXXXY BJXBOXX*LE BXXXXPOBEX XXYXXxxxxx
XLXXOKXLXZ XxxxxxXXXX BXXXXXZXXX ZXXXXXXxXX XXXBXXXXXX

401 XXXXBxxxxx xxxxXXXXXX LXXXXXXXXX XXX*xxXXXX Xxxxxxxxxx
    XXXXXXXXXX XXXUX*XXXX XXPLXXX*XJ XxXXXXXXXX XXXXXBxXXX

501 XXZXXxxxxx xx*x*XXXXX XXXXXXXxxx XXXXXXXLXX LYXXXXXXXJ
    XXXxXBXxBB ZXXXXXEXXX XXBXZXXXXX XXBXXXXBXx xxXXKxxxxx

601 XxxxxxxxxE XLUZXUXBXL XXXUXBXBXB XXXXXXXYXL K*KUPZXXXX
    XXXBXBEXXX xUXBXXXXXX XZXXXXXXZx XXXXXXYXBX ZXOxxxxxxX

701 xXLXxxxxxx xxxXUXXXXB BLEKLEBBPX X

Numbering is for convenience and approximate location only.

### Symbols used:

| | | | |
|---|---|---|---|
| A = ala | G = gly | M = met | S = ser |
| C = cys | H = his | N = asn | T = thr |
| D = asp | I = ile | P = pro | V = val |
| E = glu | K = lys | Q = gln | W = trp |
| F = phe | L = leu | R = arg | Y = tyr |
| K = K or R | | | |
| E = E or D | | | |
| L = L or I | | | |
| B = M, L, I, V, or F | | | |
| J = K, R, E, or D | | | |
| O = A or T | | | |
| U = N or Q | | | |
| Z = G or S | | | |
| X = any naturally occurring amino acid, except C. | | | |
| * = any naturally occurring amino acid. | | | |
| x = any naturally occurring amino acid, except C | | | |
| (or complete omission of any amino acids). | | | |

Where a stretch of wild-card amino acids are encountered (X(n) or x(n) where n>2), repetition of a given amino acid should be avoided. Similarly P, C, E, D, K, or R utilization should be minimized.

Formicidal toxins according to the Generic Formula of the subject invention are specifically exemplified herein by the toxin encoded by the gene designated 86Q3(a). Since this toxin is merely exemplary of the toxins represented by the Generic Formula presented herein, it should be readily apparent that the subject invention further comprises equivalent toxins (and nucleotide sequences coding for equivalent toxins) having the same or similar biological activity of 86Q3(a). These equivalent toxins will have amino acid homology with 86Q3(a). This amino acid homology will typically be greater than 50%, preferably be greater than 75%, and most preferably be greater than 90%. The amino acid homology will be highest in certain critical regions of the toxin which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Table 2 provides a listing of examples of amino acids belonging to each class.

**Table 2**

| Class of Amino Acid | Examples of Amino Acids |
|---|---|
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the biological activity of the toxin. The information presented in the generic formulae of the subject invention provides clear guidance to the person skilled in this art in making various amino acid substitutions.

Further guidance for characterizing the formicidal toxins of the subject invention is provided in Tables 4 and 5, which demonstrate the relatedness among toxins within the formicidal toxins. These tables show a numeric score for the best matching alignment between two proteins that reflects: (1) positive scores for exact matches, (2) positive or negative scores reflecting the likelihood (or not) of one amino acid substituting for another in a related protein, and (3) negative scores for the introduction of gaps. A protein sequence aligned to itself will have the highest possible score-i.e., all exact matches and no gaps. However, an unrelated protein or a randomly generated sequence will typically have a low positive score. Related sequences have scores between the random background score and the perfect match score.

The sequence comparisons were made using the local homology algorithm of Smith and Waterman ([1981] *Advances in Applied Mathematics* 2:482-489), implemented as the program "Bestfit" in the GCG Sequence Analysis Software Package Version 7 April 1991. The sequences were compared with default parameter values (comparison table: Swgappep.Cmp, Gap weight:3.0, Length weight:0.1) except that gap limits of 250 residues were applied to each sequence compared. The program output value compared is referred to as the Quality score.

Tables 4 and 5 show the pairwise alignments between the indicated amino acids of the ant-active proteins and representatives of dipteran (CryIV; ISRH3 of Sen, K. *et al.* [1988] *Agric*. *Biol. Chem.* 52:873-878), lepidopteran and dipteran (CryIIA; CryB1 of Widner and Whiteley [1989] *J. Bacteriol.* 171:965-974), and lepidopteran (CryIA(c); Adang *et al*. [1981] *Gene* 36:289-300) proteins.

Table 3 shows which amino acids were compared from the proteins of interest.

**Table 3**

| Protein | Amino acids compared |
|---|---|
| 86Q3(a) | 1-697 |
| 63B | 1-692 |
| 33F2 | 1-618 |
| 17a | 1-677 |
| 17b | 1-678 |
| CryIV | 1-633 |
| CryIIA | 1-633 |
| CryIA(c) | 1-609 |

Table 4 shows the scores prior to adjustment for random sequence scores.

**Table 4**

| | 86Q3(a) | 63B | 33F2 | 17b | 17a | CryIVA | CryIIA | CryIA(c) |
|---|---|---|---|---|---|---|---|---|
| 86Q3(a) | 1046 | 389 | 310 | 342 | 340 | 236 | 237 | 238 |
| 63B | | 1038 | 274 | 339 | 338 | 235 | 228 | 232 |
| 33F2 | | | 927 | 323 | 322 | 251 | 232 | 251 |
| 17b | | | | 1017 | 1007 | 238 | 240 | 236 |
| 17a | | | | | 1016 | 240 | 240 | 237 |
| CryIVA | | | | | | 950 | 245 | 325 |
| CryIIA | | | | | | | 950 | 244 |
| CryIA(c) | | | | | | | | 914 |

Note that ant-active protein 86Q3(a) is more closely related to 63B, 17a, 17b, and 33F2 than it is to the CryIVA, CryIIA, and CryIA(c) toxins.

Table 5 shows the same analysis after subtraction of the average score of 50 alignments of random shuffles of the column sequences with the row sequences.

**Table 5**

| | 86Q3(a) | 63B | 33F2 | 17b | 17a | CryIVA | CryIIA | CryIA(c) |
|---|---|---|---|---|---|---|---|---|
| 86Q3(a) | 841 | 184 | 118 | 136 | 135 | 41 | 40 | 50 |
| 63B | | 831 | 81 | 133 | 130 | 40 | 33 | 43 |
| 33F2 | | | 740 | 130 | 128 | 65 | 50 | 71 |
| 17b | | | | 811 | 798 | 42 | 44 | 47 |
| 17a | | | | | 808 | 43 | 44 | 44 |
| CryIVA | | | | | | 761 | 54 | 141 |
| CryIIA | | | | | | | 755 | 55 |
| CryIA(c) | | | | | | | | 729 |

Note that in Table 5 the same relationships hold as in Table 4, i.e., 86Q3(a)'s highest score, aside from itself, is with 63B.

This degree of relatedness provides the basis for using common or similar sequence elements from the previously-described known genes to obtain related, but non-identical genes from an ant-active isolate.

Thus, certain toxins according to the subject invention can be defined as those which have formicidal activity and have an alignment value (according to the procedures of Table 5) greater than 100 with 86Q3(a). As used herein, the term "alignment value" refers to the scores obtained using the methods described above which were used to create the scores reported in Table 5.

The toxins of the subject invention can also be characterized In terms of the shape and location of toxin inclusions.

### Inclusion type

PS86Q3--Long amorphic inclusion and a small inclusion, both of which remain with the spore after lysis. See Figure 3.
PS140E2--An elliptical coated inclusion situated outside the exosporium, and a long inclusion inside the exosporium. See Figure 4.
PS211B2--Large round amorphic inclusion with coat, and an elliptical inclusion. See Figure 5.

The genes and toxins according to the subject invention include not only the full length sequences disclosed herein but also fragments of these sequences, or fusion proteins, which retain the characteristic formicidal activity of the sequences specifically exemplified herein.

It should be apparent to a person skilled in this art that genes coding for ant-active toxins can be identified and obtained through several means. The specific genes may be obtained from a culture depository as described below. These genes, or portions thereof, may be constructed synthetically, for example, by use of a gene machine. Variations of these genes may be readily constructed using standard techniques for making point mutations. Also, fragments of these genes can be made using commercially available exonucleases or endonucleases according to standard procedures. For example, enzymes such as *Bal*31 or site-directed mutagenesis can be used to systematically cut off nucleotides from the ends of these genes. Also, genes which code for active fragments may be obtained using a variety of other restriction enzymes. Proteases may be used to directly obtain active fragments of these toxins.

Equivalent toxins and/or genes encoding these equivalent toxins can also be located from *B.t.* isolates and/or DNA libraries using the teachings provided herein. There are a number of methods for obtaining the ant-active toxins of the instant invention which occur in nature. For example, antibodies to the ant-active toxins disclosed and claimed herein can be used to identify and isolate other toxins from a mixture of proteins. Specifically, antibodies may be raised to the portions of the ant-active toxtins which are most constant and most distinct from other *B.t.* toxins. These antibodies can then be used to specifically identify equivalent toxins with the characteristic formicidal activity by immunoprecipitadion, enzyme linked immunoassay (ELISA), or Western blotting. Antibodies to the toxins disclosed herein, or to equivalent toxins, or fragments of these toxins, can readily be prepared using standard procedures in this art. The genes coding for these toxins can then be obtained from the microorganism.

A further method for identifying the toxins and genes of the subject invention is through the use of oligonucleotide probes. These probes are nucleotide sequences having a detectable label. As is well known in the art, if the probe molecule and nucleic acid sample hybridize by forming a strong bond between the two molecules, it can be reasonably assumed that the probe and sample are essentially identical. The probe's detectable label provides a means for determining in a known manner whether hybridization has occurred. Such a probe analysis provides a rapid method for identifying formicidal endotoxin genes of the subject invention.

The nucleotide segments which are used as probes according to the invention can be synthesized by use of DNA synthesizers using standard procedures. In the use of the nucleotide segments as probes, the particular probe is labeled with any suitable label known to those skilled in the art, including radioactive and non-radioactive labels. Typical radioactive labels include ³²P, ¹²⁵I, ³⁵S, or the like. A probe labeled with a radioactive isotope can be constructed from a nucleotide sequence complementary to the DNA sample by a conventional nick translation reaction, using a DNase and DNA polymerase. The probe and sample can then be combined in a hybridization buffer solution and held at an appropriate temperature until annealing occurs. Thereafter, the membrane is washed free of extraneous materials, leaving the sample and bound probe molecules typically detected and quantified by autoradiography and/or liquid scintillation counting.

Non-radioactive labels include, for example, ligands such as biotin or thyroxine, as well as enzymes such as hydrolases or perixodases, or the various chemiluminescers such as luciferin, or fluorescent compounds like fluorescein and its derivatives. The probe may also be labeled at both ends with different types of labels for ease of separation, as, for example, by using an isotopic label at the end mentioned above and a biotin label at the other end.

Duplex formation and stability depend on substantial complementarity between the two strands of a hybrid, and, as noted above, a certain degree of mismatch can be tolerated. Therefore, the probes of the subject invention include mutations (both single and multiple), deletions, insertions of the described sequences, and combinations thereof, wherein said mutations, insertions and deletions permit formation of stable hybrids with the target polynucleotide of interest. Mutations, insertions, and deletions can be produced in a given polynucleotide sequence in many ways, and these methods are known to an ordinarily skilled artisan. Other methods may become known in the future.

The known methods include, but are not limited to:
(1) synthesizing chemically or otherwise an artificial sequence which is a mutation, insertion or deletion of the known sequence;
(2) using a probe of the present invention to obtain via hybridization a new sequence or a mutation, insertion or deletion of the probe sequence; and
(3) mutating, inserting or deleting a test sequence *in vitro* or *in vivo*.

It is important to note that the mutational, insertional, and deletional variants generated from a given probe may be more or less efficient than the original probe. Notwithstanding such differences in efficiency, these variants are within the scope of the present invention.

Thus, mutational, insertional, and deletional variants of the disclosed test sequences can be readily prepared by methods which are well known to those skilled in the art. These variants can be used in the same manner as the instant probes so long as the variants have substantial sequence homology with the probes. As used herein, substantial sequence homology refers to homology which is sufficient to enable the variant to function in the same capacity as the original probe. Preferably, this homology is greater than 50%; more preferably, this homology is greater than 75%; and most preferably this homology is greater than 90%. The degree of homology needed for the variant to function in its intended capacity will depend upon the intended use of the sequence. It is well within the skill of a person trained in this art to make mutational, insertional, and deletional mutations which are designed to improve the function of the sequence or otherwise provide a methodological advantage.

Specific nucleotide probes useful, according to the subject invention, in the rapid identification of ant-active genes are
(i) DNA coding for a peptide sequence whose single letter amino acid designation is "REWINGAN" (SEQ ID NO. 11) or variations thereof which embody point mutations according to the following: position 1, R or K; position 3, W or Y; position 4, I or L; position 7, A or N; position 8, N or Q; a specific example of such a probe is "AGA(A or G)T(G or A)(G or T)(A or T)T(A or T)AATGG(A or T)GC(G or T)(A or C)A" (SEQ ID NO. 12); another example of such a probe is "GA(A or G)TGG(A or T)TAAATGGT(A or G)(A or C)(G or C)AA" (SEQ ID NO. 13);
(ii) DNA coding for a peptide sequence whose single letter amino acid designation is "PTFDPDLY" (SEQ ID NO. 14) or variations thereof which embody point mutations according to the following: position 3, F or L; position 4, D or Y; position 5, P or T; position 6, D or H; position 7, L or H or D or N; a specific example of such a probe is "CC(A or T)AC(C or T)TTT(T or G)ATCCAGAT(C or G)(T or A)(T or C)TAT" (SEQ ID NO. 15); another example of such a probe is "CC(T or A)AC(T or A)TT(T or C)GAT(C or A)CA(G or C)AT(C or A)(T or A)TTAT" (SEQ ID NO. 16);
(iii) additional useful probes for detecting ant-active *B.t.* genes include "GCAATTTTAA ATGAATTATA TOC" (SEQ ID NO. 23), "CAAYTACAAG CWCAACC" (SEQ ID NO. 24), "AATGAAGTWF ATCCWGTWAA T" (SEQ ID NO. 27), "GCAAGCGGCC GCTTATGGAA TAAATTCAAT TYKRTCWA" (SEQ ID NO. 28), "AGACTGGATC CATGGCWACW ATWAATGAAT TATAYCC" (SEQ ID NO. 29), "TAACGTGTAT WCGSTTTTAA TTTWGAYTC" (SEQ ID NO. 31), "TGGAATAAAT TCAATTYKRT CWA" (SEQ ID NO.33), "AGGAACAAAYTCAAKWCGRT CTA" (SEQ ID NO. 34), and "TCTCCATCTT CTGARGWAAT" (SEQ ID NO. 37).

The potential variations in the probes listed is due, in part, to the redundancy of the genetic code. Because of the redundancy of the genetic code, i.e., more than one coding nucleotide triplet (codon) can be used for most of the amino acids used to make proteins. Therefore different nucleotide sequences can code for a particular amino acid. Thus, the amino acid sequences of the *B.t.* toxins and peptides can be prepared by equivalent nucleotide sequences encoding the same amino acid sequence of the protein or peptide. Accordingly, the subject invention includes such equivalent nucleotide sequences. Also, inverse or complement sequences are an aspect of the subject invention and can be readily used by a person skilled in this art. In addition it has been shown that proteins of identified structure and function may be constructed by changing the amino acid sequence if such changes do not alter the protein secondary structure (Kaiser, E.T., Kezdy, F.J. [1984] *Science* 223:249-255). Thus, the subject invention includes mutants of the amino acid sequence depicted herein which do not alter the protein secondary structure, or if the structure is altered, the biological activity is substantially retained. Further, the invention also includes mutants of organisms hosting all or part of a toxin encoding a gene of the invention. Such microbial mutants can be made by techniques well known to persons skilled in the art. For example, UV irradiation can be used to prepare mutants of host organisms. Likewise, such mutants may include asporogenous host cells which also can be prepared by procedures well known in the art.

The toxin genes or gene fragments exemplified according to the subject invention can be obtained from *B. thuringiensis* (*B.t.*) isolates designated PS17, PS33F2, PS63B, and PS86Q3. Subcultures of the *E. coli* host harboring the toxin genes of the invention were deposited in the permanent collection of the Northern Research Laboratory, U.S. Department of Agriculture, Peoria, Illinois, USA. The accession numbers are as follows:

| Culture | Repository No. | Deposit Date |
|---|---|---|
| *B.t.* PS140E2 | NRRL B-18812 | April 23, 1991 |
| *B.t.* PS86Q3 | NRRL B-18765 | February 6, 1991 |
| *B.t.* PS211B2 | NRRL B-18921 | November 15, 1991 |
| *B.t*. PS17 | NRRL B-18243 | July 28, 1987 |
| *B.t.* PS33F2 | NRRL B-18244 | July 28, 1987 |
| *B.t.* PS63B | NRRL B-18246 | July 28, 1987 |
| *E. coli* NM522(pMYC2316)(33F2) | NRRL B-18785 | March 15, 1991 |
| *E. coli* NM522(pMYC2321) | NRRL B-18770 | February 14, 1991 |
| *E. coli* NM522(pMYC2317) | NRRL B-18816 | April 24, 1991 |
| *E. coli* NM522(pMYC1627)(17a) | NRRL B-18651 | May 11, 1990 |
| *E. coli* NM522(pMYC1628)(17b) | NRRL B-18652 | May 11, 1990 |
| *E. coli* NM522(pMYC1642)(63B) | NRRL B-18961 | April 10, 1992 |
| *E. coli* MR618(pMYC1647)(86Q3) | NRRL B-18970 | April 29, 1992 |

The subject cultures have been deposited under conditions that assure that access to the cultures will be available during the pendency of this patent application to one determined by the Commissioner of Patents and Trademarks to be entitled thereto under 37 CFR 1.14 and 35 USC 122. The deposits are available as required by foreign patent laws in countries wherein counterparts of the subject application, or its progeny, are filed. However, it should be understood that the availability of a deposit does not constitute a license to practice the subject invention in derogation of patent rights granted by governmental action.

Further, the subject culture deposits will be stored and made available to the public in accord with the provisions of the Budapest Treaty for the Deposit of Microorganisms, i.e., they will be stored with all the care necessary to keep them viable and uncontaminated for a period of at least five years after the most recent request for the furnishing of a sample of the deposit, and in any case, for a period of at least 30 (thirty) years after the date of deposit or for the enforceable life of any patent which may issue disclosing the cultures. The depositor acknowledges the duty to replace the deposits should the depository be unable to furnish a sample when requested, due to the condition of the deposit(s). All restrictions on the availability to the public of the subject culture deposits will be irrevocably removed upon the granting of a patent disclosing them.

The *B.t.* isolates of the invention can be cultured using standard art media and fermentation techniques. Upon completion of the fermentation cycle, the bacteria can be harvested by first separating the *B.t.* spores and crystals from the fermentation broth by means well known in the art. The recovered *B.t.* spores and crystals can be formulated into a wettable powder, liquid concentrate, granules, or other formulations by the addition of surfactants, dispersants, inert carriers and other components to facilitate handling and application for particular target pests. These formulation and application procedures are all well known in the art.

Formulated products can be sprayed or applied as baits to control hymenopteran pests. When applied with a bait, the *B.t.* itself may be used, or another suitable host, as described herein, may be transformed with a *B.t.* gene and used to express toxins. A vegetable oil or other liquid substance can be added to a bait to make it more attractive to the pests. Various attractants, including pheromone compounds, are well known to those skilled in the art and can be used as a component of the bait. The bait and toxin or toxin-producing microbe can be used as part of a trap.

The *B.t.* cells of the invention can be treated prior to formulation to prolong the pesticidal activity when the cells are applied to the environment of a target pest. Such treatment can be by chemical or physical means, or by a combination of chemical and/or physical means, so long as the technique does not deleteriously affect the properties of the pesticide, nor diminish the cellular capability in protecting the pesticide. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride; alcohols, such as isopropyl and ethanol; various histologic fixatives, such as Bouin's fixative and Helly's fixative (See: Humason, Gretchen. L, *Animal Tissue Techniques*, W.H. Freeman and Company, 1967); or a combination of physical (heat) and chemical agents that prolong the activity of the toxin produced in the cell when the cell is applied to the environment of the target pest(s). Examples of physical means are short wavelength radiation such as gamma-radiation and X-radiation, freezing, UV irradiation, lyophilization, and the like.

Genes encoding toxins having activity against the target susceptible pests can be isolated from the *B.t.* isolate of the invention by use of well known procedures.

The toxin genes of the subject invention can be introduced into a wide variety of microbial hosts. Expression of the toxin gene results, directly or indirectly, in the intracellular production and maintenance of the pesticide. With suitable hosts, e.g., *Pseudomonas,* the microbes can be applied to the situs of hymenopteran insects where they will proliferate and be ingested by the insects. The result is a control of the unwanted insects. Alternatively, the microbe hosting the toxin gene can be treated under conditions that prolong the activity of the toxin produced in the cell. The treated cell then can be applied to the environment of target pest(s). The resulting product retains the toxicity of the *B.t.* toxin.

Where the *B.t.* toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, e.g., *genera Pseudomonas, Erwinia, Serratia, Klebsiella, Xanthomonas, Streptomyces, Rhizobium, Rhodopseudomonas, Methylophillus, Agrobacterium, Acetobacter, Lactobacillus, Arthrobacter, Azotabacter, Leuconostoc, and Alcaligenes; fungi,* particularly yeast, e.g., genera *Saccharomyces, Cryptococcus, Kluyveromyces, Sporobolomyces, Rhodotorula,* and *Aureobasidium.* Of particular interest are such phytosphere bacterial species as *Pseudononas syringae, Pseudomonas fluorescens, Serratia marcescens, Acetobacter xylinum, Agrobacterium nomefaciens, Rhodopseudomonas spheroides, Xanthomonas campestris, Rhizobium melioti, Alcaligenes entrophus,* and *Azotobacter vinlandii* and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Khyveromyces veronae*, and *Aureobasidium pollulans*. Of particular interest are the pigmented microoganisms.

A wide variety of ways are available for introducing the *B.t.* gene expressing the toxin into the microorganism host under conditions which allow for stable maintenance and expression of the gene. One can provide for DNA constructs which include the transcriptional and translational regulatoly signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment This can be achieved with operators or a region binding to an activator or enhancers, which are capable of induction upon a change in the physical or chemical environment of the microorganisms For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger, particularly by using an active promoter, as well as by employing sequences, which enhance the stability of the messenger RNA. The initiation and translational termination region will involve stop coden(s), a terminator region, and optionally, a polyadenylation signal.

In the direction of transcription, namely in the 5' to 3' direction of the coding or sense sequence, the construct will involve the transcriptional regulatory region, If any, and the promoter, where the regulatory region may be either 5' or 3' of the promoter, the ribosomal binding site, the initiation codon, the structural gene having an open reading frame in phase with the initiation codon, the slop codon(s), the polyadenylation signal sequence, If any, and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototropy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may effectively compete with the wild-type microorganisms and stably occupy a niche in the environment.

Where no functional replication system is present, the construct will also include a sequence of at least 50 basepairs (bp), preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the *trp gene, lac* gene, *gal* gene, the lambda left and right promoters, the tac promoter, the naturally-occurring promoters associated with the toxin gene, where functional in the host. See for example U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome, an episomal element normally present in the host or a different host, or a replication system from a virus which is stable in the host. A large number of plasmids are available, such as pBR322, pACYC184, RSF1010, pRO1614, and the like. See for example, Olson *et al.* (1982) *J. Bacteriol.* 150:6069; Bagdasarian *et al.* (1981) *Gene* 16:237; and U.S. Patent Nos. 4,356,270, 4,362,817, and 4,371,625.

The *B.t.* gene can be introduced between the transcriptional and translational initiation region and the transcriptional and translational termination region, so as to be under the regulatory control of the initiation region. This construct will be included in a plasmid which will include at least one replication system, but may include more than one, where one replication system is employed for doning during the development of the plasmid and the second replication system is necessary for functioning in the ultimate host. In addition, one or more markers may be present, which have been described previously. Where integration is desired, the plasmid will desirably include a sequence homologous with the host genome.

The transformants can be isolated in accordance with conventional ways, usually employing a selection technique, which allows for selection of the desired organism as against unmodified organisms or transferring organisms, when present. The transformants then can be tested for pesticidal activity.

Suitable host cells, where the pesticide-containing cells will be treated to prolong the activity of the toxin in the cell when the then treated cell is applied to the environment of target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and -positive, include Enterobacteriaceae, such as *Ercherichia, Erwinia, Shigella, Salmonella,* and *Proteus; Bacillaceae; Rhizobiceae,* such as *Rhizobium; Spirillaceae*, such as photobacterium, *Zymomonas, Serratia, Aeromonas, Vibrio, Desulfovibrio, Spirillum;* Lactobacillaceae; Pseudomonadaceac, such as *Pseudomonas* and *Acetobacter, Azotobacteraccae* and *Nitrobacteraceae.* Among eukazyotes are fungi, such as Phycomycetes and Ascomycetes, which includes yeast, such as *Saccharomyces* and *Schizosaccharomyces;* and Basidiomycetes yeast, such as *Rhodotorula, Aureobasidium, Sporobolomyces,* and the like.

Characteristics of particular interest in selecting a host cell for purposes of production include case of introducing the *B.t.* gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as *Rhodotorula* sp., *Aureobasidium* sp., *Saccharomyces* sp., and *Sporobolomyces* sp.; phylloplane organisms such as *Pseudomonas* sp., *Erwinia* sp. and *Flavobacterium* sp.; or such other organisms as *Escherichia, Lactobacillus* sp., *Bacillus* sp., *Streptomyces* sp., and the like. Specific organisms include *Pseudomonas aeruginosa, Pseudomonas fluorescens, Saccharomyces cerevisiae, Bacillus thuringiensis Escherichia coil, Bacillus subtilis, Streptomyces lividans*, and the like.

The cell will usually be intact and be substantially in the proliferative form when treated, rather than in a spore form, although in some instances spores may be employed.

Treatment of the recombinant microbial cell can be done as disclosed *infra.* The treated cells generally will have enhanced structural stability which will enhance resistance to environmental conditions. Where the pesticide is in a proform, the method of inactivation should be selected so as not to inhibit processing of the preform to the mature form of the pesticide by the target pest pathogen. For example, formaldehyde will crosslink proteins and could inhibit processing of the proform of a polypeptide pesticide. The method of inactivation or killing retains at least a substantial portion of the bio-availability or bioactivity of the toxin.

The cellular host containing the *B.t.* insecticidal gene may be grown in any convenient nutrient medium, where the DNA construct provides a selective advantage, providing for a selective medium so that substantially all or all of the cells retain the *B.t.* gene. These cells may then be harvested in accordance with conventional ways. Alternatively, the cells can be treated prior to harvesting.

The *B.t.* cells may be formulated in a variety of ways. They may be employed as wettable powders, baits, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in as least 1% by weight and may be 100% by weight. The dry formulations will have from about 1-95% by weight of the pesticide while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the hymenopteran pest(s), e.g., plants, soil or water, by spraying, dusting, sprinkling baits or the like.

Following are examples which illustrate procedures, including the best mode, for practicing the invention. These examples should not be construed as limiting. All percentages are by weight and all solvent mixture proportions are by volume unless otherwise noted.

### Example 1 - Culturing B.t. Isolates of the Invention

A subculture of a *B.t.* isolate can be used to inoculate the following medium, a peptone, glucose, salts medium.

| | |
|---|---|
| Bacto Peptone | 7.5 g/l |
| Glucose | 1.0 g/l |
| KH₂PO₄ | 3.4 g/l |
| K₂HPO₄ | 4.35 g/l |
| Salts Solution | 5.0 ml/l |
| CaCl₂ Solution | 5.0 ml/l |

| Salts Solution (100 ml) | |
|---|---|
| MgSO₄·7H₂O | 2.46 g |
| MnSO₄·H₂O | 0.04 g |
| ZnSO₄·H₂O | 0.28 g |
| FeSO₄·7H₂O | 0.40 g |

| CaCl₂ Solution (100 ml) | |
|---|---|
| CaCl₂·2H₂O | 3.66 g |
| pH 7.2 | |

The salts solution and CaCl₂ solution are filter-sterilized and added to the autoclaved and cooked broth at the time of inoculation. Flasks are incubated at 30°C on a rotary shaker at 200 rpm for 64 hr.

### Example 2 - Purification of Protein and Amino Acid Sequencing

The *B.t.* isolates PS86Q3, PS17, PS63B, and PS33F2 were cultured as described in Example 1. The parasporal inclusion bodies were partially purified by sodium bromide (28-38%) isopycnic gradient centrifugation (Pfannenstiel, M.A., E.J. Ross, V.C. Kramer, K.W. Nickerson [1984] *FEMS Microbiol. Lett.* 21:39). The proteins were bound to PVDF membranes (Millipore, Bedford, MA) by western blotting techniques (Towbin, H., T. Staehlelin, K. Gordon [1979] *Proc. Natl. Acad. Sci. USA* 76:4350) and the N-terminal amino acid sequences were determined by the standard Edman reaction with an automated gas-phase sequenator (Hunkapiller, M.W., R.M. Hewick, W.J. Dreyer, and L.E. Hood [1983] *Meth. Enzymol.* 91:399). The sequences obtained were:
17a: A I L N E L Y P S V P Y N V (SEQ ID NO. 17)
17b: A I L N E L Y P S V P Y N V (SEQ ID NO. 18)
86Q3(a): M A T I N E L Y P N V P Y N V L (SEQ ID NO. 19)
63B: Q L Q A Q P L I P Y N V L A (SEQ ID NO. 20)
33F2: A T L N E V Y P V N (SEQ ID NO. 21)

In addition, internal amino acid sequence data were derived for 63B. The toxin protein was partially digested with *Staphylococcus aureus* V8 protease (Sigma Chem. Co., St. Louis, MO) essentially as described (Cleveland, D.W., S.G. Fischer, M.W. Kirschner, U.K. Laemmli [1977] *J. Biol. Chem.* 252:1102). The digested material was blotted onto PVDF membrane and a ca. 28 kDa limit peptide was selected for N-terminal sequencing as described above. The sequence obtained was:
63B(2) V Q R I L D E K L S F Q L I K (SEQ ID NO. 22)

From these sequence data oligonucleotide probes were designed by utilizing a codon frequency table assembled from available sequence data of other *B.t.* toxin genes. The probes were synthesized on an Applied Biosystems, Inc. DNA synthesis machine.

Protein purification and subsequent amino acid analysis of the N-terminal peptides listed above has led to the deduction of several oligonucleotide probes for the isolation of toxin genes from formicidal *B.t.* isolates. RFLP analysis of restricted total cellular DNA using radiolabeled oligonucleotide probes has elucidated different genes or gene fragments.

### Example 3 - Cloning of Novel Toxin Genes and Transformation into Escherichia coli

Total cellular DNA was prepared by growing the cells *B.t.* PS17 to a low optical density (OD₆₀₀ = 1.0) and recovering the cells by centrifugation. The cells were protoplasted In TES buffer (30 mM Tris-Cl, 10 mM EDTA, 50 mM NaCl, pH = 8.0) containing 20 % sucrose and 50 mg/ml lysozyme. The protoplasts were lysed by addition of SDS to a final concentration of 4%. The cellular material was precipitated overnight at 4°C in 100 mM (final concentration) neutral potassium chloride. The supernate was extracted twice with phenol/chloroform (1:1). The DNA was precipitated with ethanol and purified by isopycnic banding on a cesium chloride-ethidium bromide gradient.

Total cellular DNA from PS17 was digested with *Eco*RI and separated by electrophoresis on a 0.8% (w/v) Agarose-TAE (50 mM Tris-HCl, 20 mM NaOAc, 2.5 mM EDTA, pH=8.0) buffered gel. A Southern blot of the gel was hybridized with a [³²P]-radiolabeled oligonucleotide probe derived from the N-terminal amino acid sequence of purified 130 kDa protein from PS17. The sequence of the oligonucleotide synthesized is (GCAATTTTAAATGAATTATATCC) (SEQ ID NO. 23). Results showed that the hybridizing *Eco*RI fragments of PS17 are 5.0 kb, 4.5 kb, 2.7 kb and 1.8 kb in size, presumptively identifying at least four new ant-active toxin genes, 17d, 17b, 17a and 17e, respectively.

A library was constructed from PS17 total cellular DNA partially digested with *Sau*3A and size fractionated by electrophoresis. The 9 to 23 kb region of the gel was excised and the DNA was electroeluted and then concentrated using an Elutip™ ion exchange column (Schleicher and Schuel, Keene NH). The isolated *Sau*3A fragments were ligated into LambdaGEM-11™ (PROMEGA). The packaged phage were plated on KW251 *E. coli* cells (PROMEGA) at a high titer and screened using the above radiolabeled synthetic oligonucleotide as a nucleic acid hybridization probe. Hybridizing plaques were purified and rescreened at a lower plaque density. Single isolated purified plaques that hybridized with the probe were used to infect KW251 *E. coli* cells in liquid culture for preparation of phage for DNA isolation. DNA was isolated by standard procedures.

Recovered recombinant phage DNA was digested with *Eco*RI and separated by electrophoresis on a 0.8% agarose-TAE gel. The gel was Southern blotted and hybridized with the oligonucleotide probe to characterize the toxin genes isolated from the lambda library. Two patterns were present, clones containing the 4.5 kb (17b) or the 2.7 kb (17a) *Eco*RI fragments. Preparative amounts of phage DNA were digested with *Sal*I (to release the inserted DNA from lambda arms) and separated by electrophoresis on a 0.6% agarose-TAE gel. The large fragments, electroeluted and concentrated as described above, were ligated to *Sal*I-digested and dephosphorylaled pBClac, an *E. coli/B.t.* shuttle vector comprised of replication origins from pBC16 and pUC19. The ligation mix was introduced by transformation into NM522 competent *E. coli* cells and plated on LB agar containing ampicillin, isopropyl-(Beta)-D-thiogalactoside (IPTG) and 5-Bromo-4-Chloro-3-indolyl-(Beta)-D-galactoside (XGAL). White colonies, with putative insertions in the (Beta)-galactosidase gene of pBClac, were subjected to standard rapid plasmid purification procedures to isolate the desired plasmids. The selected plasmid containing the 2.7 kb *Eco*RI fragment was named pMYC1627 and the plasmid containing the 4.5 kb *Eco*RI fragment was called pMYC1628.

The toxin genes were sequenced by the standard Sanger dideoxy chain termination method using the synthetic oligonucleotide probe, disclosed above, and by "walking" with primers made to the sequence of the new toxin genes.

The PS17 toxin genes were subcloned into the shuttle vector pHT3101 (Lereclus, D. *et al.* [1989] *FEMS Microbiol. Lett.* 60:211-218) using standard methods for expression in *B.t.* Briefly, *Sal*I fragments containing the 17a and 17b toxin genes were isolated from pMYC1629 and pMYC1627, respectively, by preparative agarose gel electrophoresis, electroelution, and concentrated, as described above. These concentrated fragments were ligated into *Sal*I-cleaved and dephosphorylated pHT3101. The ligation mixtures were used separately to transform frozen, competent *E. coli* NM522. Plasmids from each respective recombinant *E. coli* strain were prepared by alkaline lysis and analyzed by agarose gel electrophoresis. The resulting subclones, pMYC2311 and pMYC2309, harbored the 17a and 17b toxin genes, respectively. These plasmids were transformed into the acrystalliferous *B.t.* strain, HD-1 *cry*B (Aronson, A., Purdne University, West Lafayette, IN), by standard electroporation techniques (Instruction Manual, Biorad, Ricmond, CA).

Recombinant *B.t.* stains HD-1 *cry*B [pMYC2311] and [pMYC2309] were grown to sporulation and the proteins purified by NaBr gradient centrifugation as described above for the wild-type *B.t.* proteins.

### Example 4 - Molecular Cloning of a Gene Encoding a Novel Toxin from Bacillus thuringiensis Strain PS63B

Example 2 shows the aminoterminal and internal polypeptide sequences of the 63B toxin protein as determined by standard Edman protein sequencing. From these sequences, two oligonucleotide primers were designed using a codon frequency table assembled from B.t. genes encoding δ-endotoxins. The sequence of the forward primer (63B-A) was complementary to the predicted DNA sequence at the 5' end of the gene:
63B-A -5' CAA T/CTA CAA GCA/F CAA CC 3' (SEQ ID NO. 24)
The sequence of the reverse primer (63B-INT) was complementary to the inverse of the internal predicted DNA sequence:
63B-INT -5' TTC ATC TAA AAT TCT TTG A/TAC 3' (SEQ ID NO. 25)
These primers were used in standard polymerase chain reactions (Cetus Corporation) to amplify an approximately 460 bp fragment of the 63B toxin gene for use as a DNA cloning probe. Standard Southern blots of total cellular DNA from 63B were hybridized with the radiolabeled PCR probe. Hybridizing bands included an approximately 4.4 kbp X*baI* fragment, an approximately 2.0 kbp *Hind*III fragment, and an approximately 6.4 kbp *Spe*I fragment.

Total cellular DNA was prepared from *Bacillus thuringiensis* (*B.t.*) cells grown to an optical density of 1.0 at 600 nm. The cells were recovered by centrifugation and protoplasts were prepared in lysis mix (300 mM sucrose, 25 mM Tris-HCl, 25 mM EDTA, pH = 8.0) and lysozyme at a concentration of 20 mg/ml. The protoplasts were ruptured by addition of ten volumes of 0.1 M NaCl, 0.1 M Tris-HCl pH 8.0, and 0.1% SDS. The cellular material was quickly frozen at -70°C and thawed to 37°C twice. The supernatant was extracted twice with phenol/chloroform (1:1). The nucleic acids were precipitated with ethanol. To remove as much RNA as possible from the DNA preparation, RNase at final concentration of 200 µg/ml was added. After incubation at 37°C for 1 hour, the solution was extracted once with phenol/chloroform and precipitated with ethanol.

A gene library was constructed from 63B total cellular DNA partially digested with *Nde*II and size fractioned by gel electrophoresis. The 9-23 kb region of the gel wan excised and the DNA was electroeluted and then concentrated using an Elutip-d ion exchange column (Schleicher and Schuel, Keene, NH). The isolated *Nde*II fragments were ligated into *Bam*HI-digested LambdaGEM-11 (PROMEGA). The packaged phage were plated on *E. coli* KW251 cells (PROMEGA) at a high titer and screened using the radiolabeled approximately 430 bp fragment probe amplified with the 63B-A and 63B internal primers (SEQ ID NOS. 27 and 28, respectively) by polymerase chain reaction. Hybridizing plaques were purified and rescreened at a lower plaque density. Single isolated, purified plaques that hybridized with the probe were used to infect KW251 cells in liquid culture for preparation of phage for DNA isolation. DNA was isolated by standard procedures (Maniatis, T., E.F. Fritsch, J. Sambrook [1982] *Molecular Cloning: A Laboratory Manual,* Cold Spring Harbor Laboratory, New York). Preparative amounts of DNA were digested with *Sal*I (to release the inserted DNA from lambda sequences) and separated by electrophoresis on a 0.6% agarose-TAE gel. The large fragments were purified by ion exchange chromatography as above and ligated to *Sal*I-digested, dephosphorylated pHTBlueII (an *E. coli/B.t.* shuttle vector comprised of pBlueScript S/K [Stratagene, San Diego, CA] and the replication origin from a resident *B.t.* plasmid [Lereclus, D. *et al*. (1989) *FEMS Microbiol. Lett.* 60:211-218]). The ligation mix was introduced by transformation into competent *E. coli* NM522 cells (ATCC 47000) and plated on LB agar containing ampicillin (100 µg/ml), IPTG (2%), and XGAL (2%). White colonies, with putative restriction fragment insertions in the (Beta)-galactosidase gene of pHTBlueII, were subjected to standard rapid plasmid purification procedures (Maniatis *et al., supra*). Plasmids ere analyzed by *Sal*I digestion and agarose gel electrophoresis. The desired plasmid construct, pMYC1641, contains an approximately 14 kb *Sal*I insert.

For subcloning, preparative amounts of DNA were digested with *Xba*I and electrophoresed on an agarose gel. The approximately 4.4 kbp band containing the toxin gene was excised from the gel, electroeluted from the gel slice, and purified by ion exchange chromatography as above. This fragment was ligated into *Xba*I cut pHTBlueII and the resultant plasmid was designated pMYC1642.

### Example 5 - Cloning of a Novel Toxin Gene From B.t. PS33F2 and Transformation into Escherichia coli

Total cellular DNA was prepared front *B.t.* PS33F2 cells grown to an optical density, at 600 nm, of 1.0. Cells were pelleted by centrifugation and resuspended in protoplast buffer (20 mg/ml lysozyme in 0.3 M sucrose, 25 mM Tris-Cl [pH 8.0], 25 mM EDTA). After incubation at 37°C for 1 hour, protoplasts were lysed by the addition of nine volumes of a solution of 0.1 M NaCl, 0.1% SDS, 0.1 M Tris-Cl followed by two cycles of freezing and thawing. The cleared lysate was extracted twice with phenol:chloroform (1:1). Nucleic acids were precipitated with two volumes of ethanol and pelleted by centrifugation. The pellet was resuspended in 10 mM Tris-Cl, 1 mM EDTA (TE) and RNase was added to a final concentration of 50 µg/ml. After incubation at 37°C for 1 hour, the solution was extracted once each with phenol:chloroform (1:1) and TE-saturated chloroform. DNA was precipitated from the aqueous phase by the addition of one-tenth volume of 3M NaOAc and two volumes of ethanol. DNA was pelleted by centrifugation, washed with 70% ethanol, dried, and resuspended in TE.

Plasmid DNA was extracted from protoplasts prepared as described above. Protoplasts were lysed by the addition of nine volumes of a solution of 10 mM Tris-Cl, 1 mM EDTA, 0.085 N NaOH, 0.1% SDS, pH=8.0. SDS was added to 1% final concentration to complete lysis. One-half volume of 3 M KOAc was then added and the cellular material was precipitated overnight at 4°C. After centrifugation, the DNA was precipitated with ethanol and plasmids were purified by isopycnic centrifugation on cesium chloride-ethidium bromide gradients.

Restriction Fragment Length Polymorphism (RFLP) analyses were performed by standard hybridization of Southern blots of PS33F2 plasmid and total cellular DNA with ³²P-labelled oligonucleotide probes designed to the N-terminal amino acid sequence disclosed in Example 2.
Probe 33F2A: 5' GCA/T ACA/T TTA AAT GAA GTA/T TAT 3' (SEQ ID NO. 26)
Probe 33F2B: 5' AAT GAA GTA/T TAT CCA/T GTA/T AAT 3' (SEQ ID NO. 27)
Hybridizing bands included an approximately 5.85 kbp *Eco*RI fragment. Probe 33F2A and a reverse PCR primer were used to amplify a DNA fragment of approximately 1.8 kbp for use as a hybridization probe for cloning the 33F2 toxin gene. The sequence of the reverse primer was:
5' GCAAGCGGCCGCTTATGGAATAAATTCAATT C/T T/G A/G TC T/A A 3' (SEQ ID NO. 28).

A gene library was constructed from 33F2 plasmid DNA digested with *Eco*RI. Restriction digests were fractionated by agarose gel electrophoresis. DNA fragments 4.3-6.6 kbp were excised from the gel, electroeluted from the gel slice, and recovered by ethanol precipitation after purification on an Elutip-D ion exchange column (Schleicher and Schuel, Keene NH). The *Eco*RI inserts were ligated into *Eco*RI-digested pHTBlueII (an *E. coli/B. thuringiensis* shuttle vector comprised of pBluescript S/K [Stratagene] and the replication origin from a resident *B.t.* plasmid (Lereclus, D. *et al.* [1989] *FEMS Microbial. Lett.* 60:211-218]). The ligation mixture was transformed into frozen, competent NM522 cells (ATCC 47000). Transformants were plated on LB apar containing ampicillin, isopropyl-(Beta)-D-thiogalactoside (IPTG), and 5-bromo-4-chloro-3-indolyl-(Beta)-D-galactoside (XGAL). Colonies were screened by hybridization with the radiolabeled PCR amplified probe described above. Plasmids were purified from putative toxin gene clones by alkaline lysis and analyzed by agarose gel electrophoresis of restriction digests. The desired plasmid construct, pMYC2316, contains an approximately 5.85 kbp *Eco*4RI insert; the toxin gene residing on this DNA fragment (33F2a) is novel compared to the DNA sequences of other toxin genes encoding formicidal proteins.

Plasmid pMYC2316 was introduced into the acrystalliferous (Cry-) *B.t.* host, HD-1 CryB (A. Aronson, Purdue University, West Lafayette, IN) by electroporation. Expression of an approximately 120-140 kDa crystal protein was verified by SDS-PAGE analysis. Crystals were purified on NaBr gradients (M.A. Pfannenstiel *et al.* [1984] *FEMS Microbiol. Lett.* 21:39) for determination of toxicity of the cloned gene product to *Pratylenchus spp.*

### Example 6 - Cloning of a Novel Toxin Gene from B.t. Isolate PS86O3

Total cellular DNA was prepared from *Bacillus thuringiensis* (*B.t.*) cells grown to an optical density of 1.0 at 600 nm. The cells were recovered by centrifugation and protoplasts were prepared in lysis mix (300 mM sucrose, 25 mM Tris-HCl, 25 mM EDTA, pH = 8.0) containing lysozyme at a concentration of 20 mg/ml The protoplasts were ruptured by addition of ten volumes of 0.1 M NaCl, 0.1% SDS, 0.1 M Tris-Cl, pH = 8.0. The cleared lysate was quickly frozen at -70°C and thawed to 37°C twice. The supernate was extracted twice with phenol:chloroform (1:1). The pellet was resuspended in 10 mM Tris-Cl, 1 mM EDTA, pH = 8.0 (TE), and RNase was added to a final concentration of 50 µg/ml. After incubation at 37°C for one hour, the solution was extracted once with phenol:chloroform (1:1) and then with TE-saturated chloroform. DNA was precipitated from the aqueous phase by the addition of one-tenth volume of 3M NaOAc and two volumes of ethanol DNA was pelleted by centrifugation, washed with 70% ethanol, dried, and resuspended in TE.

Total cellular DNA from isolate PS86Q3 was used as template for polymerase chain reaction (PCR) analysis according to protocols furnished by Perkin Elmer Cetus. An oligonucleotide derived from the N-terminal amino acid sequence of the toxin protein was used as a 5' primer. The sequence of this oligonucleotide is:
5' AGACTGGATCCATGGC(A or T)AC(A or T)AT(A or T)AATGAATTATA (T or C)CC-3' (SEQ ID NO. 29).

An oligonucleotide coding for the amino acid sequence "ESKLKPNTRY" (SEQ ID NO. 30) can be used as the reverse 3' primer. The sequence of this oligonucleotide can be: "5'-TAACGTGTAT(A or T)CG(C or G)TTTTAATTT(T or A)GA(C or T)TC-3'" (SEQ ID NO. 31).

The reverse "YIDKIEFIP" (SEQ ID NO. 32) oligonucleotide was also used as a reverse 3' primer in conjunction with the above mentioned 5' primer. The sequence of the reverse primer can be: "5'-TGGAATAAATTCAATT(C or T)(T or G)(A or G)TC(T or A)A-3'" (SEQ ID NO. 33).

Amplification with the 5' primer and SEQ ID NO. 31 generates an approximately 2.3 kbp DNA fragment and an approximately 4.3 kbp DNA fragment. Amplification with the 5' primer and SEQ ID NO. 33 generates an approximately 1.8 kbp DNA fragment and an approximately 3.7 kbp DNA fragment. The approximately 2.3 kbp fragment was radiolabeled with ³²P and used as a hybridization probe to generate restriction fragment polymorphism (RFLP) patterns and to screen recombinant phage libraries.

A Southern blot of total cellular DNA digested with *Eco*RV was probed with the radiolabeled 2.3 kbp probe described above. The resultant RFLP includes 9.5 kbp, 6.4 kbp, and 4.5 kbp hybridizing fragments.

A gene library was constructed from PS86Q3 total cellular DNA partially digested with *Nde*II and size fractioned by gel electrophoresis. The 9-23 kb region of the gel was excised and the DNA was electroeluted and then concentrated using an Elutip-d ion exchange column (Schleicher and Schuel, Keene; NH). The isolated *Nde*II fragments were ligated into *Bam*HI-digested LambdaGEM-11 (PROMEGA). The packaged phage were plated on *E. coli* KW251 cells (PROMEGA) at a high titer and screened using the radiolabeled probe described above. Hybridizing plaques were purified and rescreened at a lower plaque density. Single isolated, purified plaques that hybridized with the probe were used to infect KW251 cells in liquid culture for preparation of phage for DNA isolation. DNA was isolated by standard procedures (Maniatis *et al., supra*). Preparative amounts of DNA were digested with *Sal*I (to release the inserted DNA from lambda sequences) and separated by electrophoresis on a 0.6% agarose-TAE gel. The large fragments were purified by ion exchange chromatography as above and ligated to *Sal*I-digested, dephosphorylated pHTBlueII (an *E. coli/B.t.* shuttle vector comprised of pBluescript S/K [Stratagene, San Diego, CA]) and the replication origin from a resident *B.t.* plasmid (Lereclus *et al.* [1989], *supra*). The ligation mix was introduced by transformation into competent *E. coli* NM522 cells (ATCC 47000) and plated on LB agar containing ampicillin, IPTG, and XGAL. White coIonies, with putative restriction fragment insertions in the (Beta)-galactosidase gene of pHTBlueII were subjected to standard rapid plasmid purification procedures (Maniatis *et al*., *supra*). Plasmid DNA was analyzed by *Sal*I digestion and agarose gel electrophoresis. The desired plasmid construct, pMYC1647, contains an approximately 12 kb *Sal*I insert.

Plasmid pMYC1647 was introduced by electroporation into an acrystalliferous (Cry⁻)*B.t.*, HD-1 CryB (A.I. Aronson, Purdue University) host to yield MR515, a recombinant *B.t.* clone of 86Q3(a). Expression of an approximately 155 kDa protein was verified by SDS-PAGE. Spores and crystals were removed from broth cultures and were used for determination of toxicity to pharaoh ants.

### Example 7 - Activity of the B.t. Toxin Protein and Gene Product Against Ants

Broths were tested for the presence of β-exotoxin by a larval home fly bioassay (Campbell, D.P., Dieball, D.E., Bracket, J.M. [1987] "Rapid HPLC assay for the β-exotoxin of *Bacillus thuringiensis,*" *J. Agric. Food Chem.* 35:156-158). Only isolates which tested flee of β-exotoxin were used in the assays against ants.

A bait was made consisting of 10% *Bacillus thuringiensis* isolates of the invention and Crosse and Blackwell mint apple jelly. Approximately 100 ants were placed in each plastic test chamber replicate with the baits. Control experiments were performed with untreated mint apple jelly. Each test was replicated a minimum of 10 times. Mortality was assessed at 7,14 and 21 days after introduction of the bait to the ants. Results are shown below:

**Table 6**

| Toxicity of *B. thuringiensis* Isolates to the Pharaoh Ant (*Monomorium pharaonis*) | |
|---|---|
| *B.t.* Isolate | Percent Mortality |
| PS140E2 | 91 |
| PS 86Q3 | 84 |
| Control | 11 |
| PS211B2 | 90.0 |
| Control | 3.8 |

### Example 8 - Activity Against Pharaoh Ants

Mint apple jelly containing 10% *B.t.* (100,000 ppm) was fed to 5 replicates of approximately 100 worker ants for 21 days. Total mortality (in %) over the test period is compared to control.

**Table 7**

| Three week mortality (%) on pharaoh ant workers. | | |
|---|---|---|
| Sample | Rate ppm | Percent Mortality |
| MR515 | 100000 | 40.1 |
| 86Q3 | 100000 | 29.2 |
| 211B2 | 100000 | 58.5 |
| MAJ | Blank | 25.0 |
| Control | Blank | 14.4 |

| | | |
|---|---|---|
| MR515 = a recombinant *B.t.* clone of 86Q3(a) gene, 10% in MAJ (Example 6) | | |
| 86Q3 = spray dried powder of *B.t.* PS86Q3, 10% in MAJ | | |
| 211B2 = spray dried power of *B.t.* PS211B2, 10% in MAJ | | |
| MAJ = Mint apple jelly, Crosse & Blackwell | | |
| Control = rearing diet of water, frozen flies, mealworms/honey agar | | |

**Table 8**

| Three week mortality (%) on pharaoh ant workers. | | |
|---|---|---|
| Sample | Rate ppm | Percent Mortality |
| 140E2 | 50000 | 100.0 |
| 86Q3 | 50000 | 99.6 |
| 211B2 | 50000 | 100.0 |
| MAJ | Blank | 75.3 |
| Control | Blank | 39.0 |

| | | |
|---|---|---|
| 140E2 = 5% 140E2 purified protein in MAJ | | |
| 86Q3 = 5% 86Q3 purified protein in MAJ | | |
| 211B2 = 5% 211B2 purified protein in MAJ | | |
| MAJ = Mint apple jelly, Crosse & Blackwell | | |
| Control = rearing diet of water, frozen flies, mealworms/honey agar | | |

### Example 9 - Cloning of Novel Ant-Active Genes Using Generic Oligonucleotide Primers

The formicidal gene of a new formicidal *B.t.* can be obtained from DNA of the strain by performing the standard polymerase chain reaction procedure as in Example 6 using the oligonuclcotides of SEQ ID NO.33 or AGGAACAAAYTCAAKWCGRTCTA (SEQ ID NO. 34) as reverse primers and SEQ ID NO. 12, SEQ ID NO. 13, SEQ ID NO. 15, SEQ ID NO. 16, SEQ ID NO. 23, SEQ ID NO. 27, SEQ ID NO. 29, or SEQ ID NO. 24 as forward primers. The expected PCR fragments would be approximately 330 to 600 bp with either reverse primer and SEQ ID NO.12 or SEQ ID NO. 13, 1000 to 1400 bp with either reverse primer and SEQ ID NO. 15 or SEQ ID NO. 16, and 1800 to 2100 bp with either reverse primer and any of the three N-terminal primers, SEQ ID NO. 27, SEQ ID NO. 23, SEQ ID NO. 29, and SEQ ID NO. 24. Alternatively, a complement from the primer family described by SEQ ID NO. 12 and SEQ ID NO. 13 can be used as reverse primer with SEQ ID NO. 15, SEQ ID NO. 16, SEQ ID NO. 23, SEQ ID NO. 27, SEQ ID NO. 29, or SEQ ID NO. 24 as forward primers. The expected PCR fragments would be approximately 650 to 1000 bp with SEQ ID NO. 15 or SEQ ID NO. 16, and 1400 to 1800 bp for the four N-terminal primers (SEQ ID NO. 27, SEQ ID NO. 23, SEQ ID NO. 29, and SEQ ID NO. 24).

As another alternative, the reverse primer SEQ ID NO. 31 can be used with any of the four N-terminal forward primers to yield fragments of approximately 2550-3100 bp; 1750-2150 bp with the forward primers SEQ ID NOS. 15 or 16, 850-1400 bp with SEQ ID NOS. 12 or 13; and 550-1050 bp with the forward primer TTTAGATCGT(A or C)TTGA(G or A)TTT(A or G)T(A or T)CC(SEQ ID NO. 35).

As yet another alternative, the ITSED (SEQ ID NO 37) reverse primer (TCTCCATCTTCTGA(G or A)G(T or A)AAT) (SEQ ID NO. 37) can be used with the N-terminal forward primers (SEQ ID NO. 23, SEQ ID NO. 24, SEQ ID NO. 27, and SEQ ID NO. ID NOS. 15 or 16; 1800-2400 bp with forward primers SEQ ID NOS. 12 or 13; and 1500-2050 bp with forward primer SEQ ID NO. 35.

Amplified DNA fragments of the indicated sizes can be radiolabeled and used as probes to clone the entire gene as in Example 6.

### Example 10 - Insertion of Toxin Gene Into Plants

One aspect of the subject invention is the transformation of plants with genes coding for a formicidal toxin. The transformed plants are resistant to attack by ants.

Genes coding for formicidal toxins, as disclosed herein, can be inserted into plant cells using a variety of techniques which are well known in the art. For example, a large number of cloning vectors comprising a replication system in *E. coli* and a marker that permits selection of the transformed cells are available for preparation for the insertion of foreign genes into higher plants. The vectors comprise, for example, pBR322, pUC series, M13mp series, pACYC184, etc. Accordingly, the sequence coding for the *B.t.* toxin can be inserted into the vector at a suitable restriction site. The resulting plasmid is used for transformation into *E. coli.* The *E. coli* cells are cultivated in a suitable nutrient medium, then harvested and lysed. The plasmid is recovered. Sequence analysis, restriction analysis, electrophoresis, and other biochemical-molecular biological methods are generally carried out as methods of analysis. After each manipulation, the DNA sequence used can be cleaved and joined to the next DNA sequence. Each plasmid sequence can be cloned in the same or other plasmids. Depending on the method of inserting desired genes into the plant, other DNA sequences may be necessary. If, for example, the Ti or Ri plasmid is used for the transformation of the plant cell, then at least the right border, but often the right and the left border of the Ti or Ri plasmid T-DNA, has to be joined as the flanking region of the genes to be inserted.

The use of T-DNA for the transformation of plant cells has been intensively researched and sufficiently described in EP 120 516; Hoekema (1985) In: *The Binary Plant Vector System*, Offset-durkkerij Kanters B.V., Alblasserdam, Chapter 5; Fraley *et al.*, *Crit. Rev. Plant Sci.* 4:1-46; and An *et al.* (1985) *EMBO J.* 4:277-287.

Once the inserted DNA has been integrated in the genome, it is relatively stable there and, as a rule, does not come out again. It normally contains a selection marker that confers on the transformed plant cells resistance to a biocide or an antibiotic, such as kanamycin, G 418, bleomycin, hygromycin, or chloramphenicol, *inter alia*. The individually employed marker should accordingly permit the selection of transformed cells rather than cells that do not contain the inserted DNA.

A large number of techniques are available for inserting DNA into a plant host cell. Those techniques include transformation with T-DNA using *Agrobacterium tumefaciens* or *Agrobacterium rhizogenes* as transformation agent, fusion, injection, or electroporation as well as other possible methods. If agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the vir region necessary for the transfer of the T-DNA. Intermediate vectors cannot replicate themselves in agrobacteria. The intermediate vector can be transferred into *Agrobacterium tumefaciens* by means of a helper plasmid (conjugation). Binary vectors can replicate themselves both in *E. coli* and in agrobacteria. They comprise a selection marker gene and a linker or polylinker which are framed by the right and left T-DNA border regions. They can be transformed directly into agrobacteria (Holsters *et al.* [1978] *Mol. Gen. Genet.* 163:181-187). The agrobacterium used as host cell is to comprise a plasmid carrying a vir region. The vir region is necessary for the transfer of the T-DNA into the plant call. Additional T-DNA may be contained. The bacterium so transformed is used for the transformation of plant calls. Plant explants can advantageously be cultivated with *Agrobacterium tumefaciens* or *Agrobacterium rhizogenes* for the transfer of the DNA into the plant cell. Whole plants can then be regenerated from the infected plant material (for example, pieces of leaf, segments of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells grow inside the plants in the usual manner. They can form germ cells and transmit the transformed trait(s) to progeny plants. Such plants can be grown In the normal manner and crossed with plants that have the same transformed hereditary factors or other hereditary factors. The resulting hybrid individuals have the corresponding phenotypic properties.

### Example 11 - Cloning of Novel B. thuringiensis Genes Into Insects Viruses

A number of viruses are known to infect insects. These viruses include, for example, baculoviruses and entomopoxviruses. In one embodiment of the subject invention, ant-active genes, as described herein, can be placed with the genome of the insect virus, thus enhancing the pathogenicity of the virus. Methods for constructing insect viruses which comprise *B.t.* toxin genes are well known and readily practiced by those skilled in the art. These procedures are described, for example, in Merryweather *et al.* (Merryweather, A.T., U. Weyer, M.P.G. Harris, M. Hirst, T. Booth, R.D. Possee (1990) *J. Gen. Virol. 71*:1535-1544) and Martens *et al.* (Martens, J.W.M., G. Honee, D. Zuidema, J.W.M van Lent, B. Visser, J.M. Vlak (1990) *Appl. Environmental Microbiol. 56*(9):2764-2770).

It should be understood that the examples and embodiments described herein are for illustrative purposes only and that various modifications or changes in light thereof will be suggested to persons skilled in the art and are to be included within the spirit and purview of this application and the scope of the appended claims.

## Claims

1. A substantially pure toxin which is a protein toxic to ants and which is immunoreactive with an antibody which immunoreacts with a toxin expressed by PS140E2 or PS211B2.

2. The toxin according to claim 1, which is expressed by PS140E2.

3. The toxin according to claim 1, which is expressed by PS211B2.

4. A polynucleotide encoding a toxin as defined in claim 1.

5. The polynucleotide according to claim 4, which encodes a toxin expressed by PS140E2.

6. The polynucleotide according to claim 4, which encodes a toxin expressed by PS211B2.

7. A host comprising a nucleotide sequence which encodes a toxin as defined in claim 1.

8. The host according to claim 7, which is a *Bacillus thuringiensis.*

9. The host according to claim 8, which has the characteristics of PS140E2.

10. The host according to claim 8, which has the characteristics of PS211B2.

11. The host according to claim 7, wherein the nucleotide sequence is a heterologous sequence which has been transformed into said host and wherein said heterologous sequence is expressed at sufficient levels to result in the production of said ant toxin.

12. The host according to claim 11, which is capable of inhabiting the phylloplane or rhizosphere of a plant or is capable of survival in a baited trap.

13. A process for controlling ants, which comprises contacting the ants with a toxin as defined in claim 1.

14. A formicidal composition comprising substantially intact cells which express a toxin as defined in claim 1.

15. The composition according to claim 14, wherein the cells have been treated to prolong their formicidal activity.

16. A biologically pure culture of *Bacillus thuringiensis* PS140E2, having the identifying characteristic of activity against hymenopteran pests of NRRL B- 18812, or a mutant thereof.

17. A biologically pure culture of *Bacillus thuringiensis* PS211B2, having the identifying characteristic of activity against hymenopteran pests of NRRL B- 18921, or a mutant thereof.


SEQUENCE LISTING

(1) GENERAL INFORMATION:

(i) APPLICANT: Payne, Jewel M.
Kennedy, M. Keith
Randall, John Brooks
Meier, Henry
Uick, Heidi Jane
Foncerrada, Luis
Schnepf, Harry E.
Schwab, George E.

(ii) TITLE OF INVENTION: Novel Bacillus thuringiensis Isolates
Active Against Hymenopteran Pests and Genes Encoding
Hymenopteran-Active Toxins

(iii) NUMBER OF SEQUENCES: 38

(iv) CORRESPONDENCE ADDRESS:
(A) ADDRESSEE: David R. Saliwanchik
(B) STREET: 2421 N.W. 41st Street, Suite A-1
(C) CITY: Gainesville
(D) STATE: FL
(E) COUNTRY: USA
(F) ZIP: 32606

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: US
(B) FILING DATE:
(C) CLASSIFICATION:

(viii) ATTORNEY/AGENT INFORMATION:
(A) NAME: Saliwanchik, David R.
(B) REGISTRATION NUMBER: 31,794
(C) REFERENCE/DOCKET NUMBER: M/SCJ 104

(ix) TELECOMMUNICATION INFORMATION:
(A) TELEPHONE: 904-375-8100
(B) TELEFAX: 904-372-5800

(2) INFORMATION FOR SEQ ID NO:1:

(i) SEQUENCE CHARACTERISTICS:
(A) LENGTH: 4155 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Bacillus thuringiensis
(B) STRAIN: PS17
(C) INDIVIDUAL ISOLATE: PS17a

(vii) IMMEDIATE SOURCE:
(B) CLONE: E. coli NM522(pMYC1627) NRRL B-18651

(xi) SEQUENCE DESCRIPTION: SEQ ID NO:1:

ATGGCAATTT TAAATGAATT ATATCCATCT GTACCTTATA ATGTATTGGC GTATACGCCA 60

CCCTCTTTTT TACCTGATGC GGGTACACAA GCTACACCTG CTGACTTAAC AGCTTATGAA 120

CAATTGTTGA AAAATTTAGA AAAAGGGATA AATGCTGGAA CTTATTCGAA AGCAATAGCT 180

GATGTACTTA AAGGTATTTT TATAGATGAT ACAATAAATT ATCAAACATA TGTAAATATT 240

GGTTTAAGTT TAATTACATT AGCTGTACCG GAAATTGGTA TTTTTACACC TTTCATCGGT 300

TTGTTTTTTG CTGCATTGAA TAAACATGAT GCTCCACCTC CTCCTAATGC AAAAGATATA 360

TTTGAGGCTA TGAAACCAGC GATTCAAGAG ATGATTGATA GAACTTTAAC TGCGGATGAG 420

CAAACATTTT TAAATGGGGA AATAAGTGGT TTACAAAATT TAGCAGCAAG ATACCAGTCT 480

ACAATGGATG ATATTCAAAG CCATGGAGGA TTTAATAAGG TAGATTCTGG ATTAATTAAA 540
AAGTTTACAG ATGAGGTACT ATCTTTAAAT AGTTTTTATA CAGATCGTTT ACCTGTATTT 600
ATTACAGATA ATACAGCGGA TCGAACTTTG TTAGGTCTTC CTTATTATGC TATACTTGCG 660
AGCATGCATC TTATGTTATT AAGAGATATC ATTACTAAGG GTCCGACATG GGATTCTAAA 720
ATTAATTTCA CACCAGATGC AATTGATTCC TTTAAAACCG ATATTAAAAA TAATATAAAG 780
CTTTACTCTA AAACTATTTA TGACGTATTT CAGAAGGGAC TTGCTTCATA CGGAACGCCT 840
TCTGATTTAG AGTCCTTTGC AAAAAAACAA AAATATATTG AAATTATGAC AACACATTGT 900
TTAGATTTTG CAAGATTGTT TCCTACTTTT GATCCAGATC TTTATCCAAC AGGATCAGGT 960
GATATAAGTT TACAAAAAAC ACGTAGAATT CTTTCTCCTT TTATCCCTAT ACGTACTGCA 1020
GATGGGTTAA CATTAAATAA TACTTCAATT GATACTTCAA ATTGGCCTAA TTATGAAAAT 1080
GGGAATGGCG CGTTTCCAAA CCCAAAAGAA AGAATATTAA AACAATTCAA ACTGTATCCT 1140
AGTTGGAGAG CGGGACAGTA CGGTGGGCTT TTACAACCTT ATTTATGGGC AATAGAAGTC 1200
CAAGATTCTG TAGAGACTCG TTTGTATGGG CAGCTTCCAG CTGTAGATCC ACAGGCAGGG 1260
CCTAATTATG TTTCCATAGA TTCTTCTAAT CCAATCATAC AAATAAATAT GGATACTTGG 1320
AAAACACCAC CACAAGGTGC GAGTGGGTGG AATACAAATT TAATGAGAGG AAGTGTAAGC 1380
GGGTTAAGTT TTTTACAACG AGATGGTACG AGACTTAGTG CTGGTATGGG TGGTGGTTTT 1440
GCTGATACAA TATATAGTCT CCCTGCAACT CATTATCTTT CTTATCTCTA TGGAACTCCT 1500
TATCAAACTT CTGATAACTA TTCTGGTCAC GTTGGTGCAT TGGTAGGTGT GAGTACGCCT 1560
CAAGAGGCTA CTCTTCCTAA TATTATAGGT CAACCAGATG AACAGGGAAA TGTATCTACA 1620
ATGGGATTTC CGTTTGAAAA AGCTTCTTAT GGAGGTACAG TTGTTAAAGA ATGGTTAAAT 1680
GGTGCGAATG CGATGAAGCT TTCTCCTGGG CAATCTATAG GTATTCCTAT TACAAATGTA 1740
ACAAGTGGAG AATATCAAAT TCGTTGTCGT TATGCAAGTA ATGATAATAC TAACGTTTTC 1800
TTTAATGTAG ATACTGGTGG AGCAAATCCA ATTTTCCAAC AGATAAACTT TGCATCTACT 1860
GTAGATAATA ATACGGGAGT ACAAGGAGCA AATGGTGTCT ATGTAGTCAA ATCTATTGCT 1920
ACAACTGATA ATTCTTTTAC AGAAATTCCT GCGAAGACGA TTAATGTTCA TTTAACCAAC 1980
CAAGGTTCTT CTGATGTCTT TTTAGACCGT ATTGAATTTA TACCTTTTTC TCTACCTCTT 2040
ATATATCATG GAAGTTATAA TACTTCATCA GGTGCAGATG ATGTTTTATG GTCTTCTTCA 2100
AATATGAATT ACTACGATAT AATAGTAAAT GGTCAGGCCA ATAGTAGTAG TATCGCTAGT 2160
TCTATGCATT TGCTTAATAA AGGAAAAGTG ATAAAAACAA TTGATATTCC AGGGCATTCG 2220
GAAACCTTCT TTGCTACGTT CCCAGTTCCA GAAGGATTTA ATGAAGTTAG AATTCTTGCT 2280
GGCCTTCCAG AAGTTAGTGG AAATATTACC GTACAATCTA ATAATCCGCC TCAACCTAGT 2340
AATAATGGTG GTGGTGATGG TGGTGGTAAT GGTGGTGGTG ATGGTGGTCA ATACAATTTT 2400
TCTTTAAGCG GATCTGATCA TACGACTATT TATCATGGAA AACTTGAAAC TGGGATTCAT 2460
GTACAAGGTA ATTATACCTA TACAGGTACT CCCGTATTAA TACTGAATGC TTACAGAAAT 2520
AATACTGTAG TATCAAGCAT TCCAGTATAT TCTCCTTTTG ATATAACTAT ACAGACAGAA 2580
GCTGATAGCC TTGAGCTTGA ACTACAACCT AGATATGGTT TTGCCACAGT GAATGGTACT 2640
GCAACAGTAA AAAGTCCTAA TGTAAATTAC GATAGATCAT TTAAACTCCC AATAGACTTA 2700
CAAAATATCA CAACACAAGT AAATGCATTA TTCGCATCTG GAACACAAAA TATGCTTGCT 2760
CATAATGTAA GTGATCATGA TATTGAAGAA GTTGTATTAA AAGTGGATGC CTTATCAGAT 2820
GAAGTATTTG GAGATGAGAA GAAGGCTTTA CGTAAATTGG TGAATCAAGC AAAACGTTTG 2880
AGTAGAGCAA GAAATCTTCT GATAGGTGGG AGTTTTGAAA ATTGGGATGC ATGGTATAAA 2940
GGAAGAAATG TAGTAACTGT ATCTGATCAT GAACTATTTA AGAGTGATCA TGTATTATTA 3000
CCACCACCAG GATTGTCTCC ATCTTATATT TTCCAAAAAG TGGAGGAATC TAAATTAAAA 3060
CCAAATACAC GTTATATTGT TTCTGGATTC ATCGCACATG GAAAAGACCT AGAAATTGTT 3120

GTTTCACGTT ATGGGCAAGA AGTGCAAAAG GTCGTGCAAG TTCCTTATGG AGAAGCATTC 3180
CCGTTAACAT CAAATGGACC AGTTTGTTGT CCCCCACGTT CTACAAGTAA TGGAACCTTA 3240
GGAGATCCAC ATTTCTTTAG TTACAGTATC GATGTAGGTG CACTAGATTT ACAAGCAAAC 3300
CCTGGTATTG AATTTGGTCT TCGTATTGTA AATCCAACTG GAATGGCACG CGTAAGCAAT 3360
TTGGAAATTC GTGAAGATCG TCCATTAGCA GCAAATGAAA TACGACAAGT ACAACGTGTC 3420
GCAAGAAATT GGAGAACCGA GTATGAGAAA GAACGTGCGG AAGTAACAAG TTTAATTCAA 3480
CCTGTTATCA ATCGAATCAA CGGATTGTAT GAAAATGGAA ATTGGAACGG TTCTATTCGT 3540
TCAGATATTT CGTATCAGAA TATAGACGCG ATTGTATTAC CAACGTTACC AAAGTTACGC 3600
CATTGGTTTA TGTCAGATAG ATTCAGTGAA CAAGGAGATA TAATGGCTAA ATTCCAAGGT 3660
GCATTAAATC GTGCGTATGC ACAACTGGAA CAAAGTACGC TTCTGCATAA TGGTCATTTT 3720
ACAAAAGATG CAGCTAATTG GACAATAGAA GGCGATGCAC ATCAGATAAC ACTAGAAGAT 3780
GGTAGACGTG TATTGCGACT TCCAGATTGG TCTTCGAGTG TATCTCAAAT GATTGAAATC 3840
GAGAATTTTA ATCCAGATAA AGAATACAAC TTAGTATTCC ATGGGCAAGG AGAAGGAACG 3900
GTTACGTTGG AGCATGGAGA AGAAACAAAA TATATAGAAA CGCATACACA TCATTTTGCG 3960
AATTTTACAA CTTCTCAACG TCAAGGACTC ACGTTTGAAT CAAATAAAGT GACAGTGACC 4020
ATTTCTTCAG AAGATGGAGA ATTCTTAGTG GATAATATTG CGCTTGTGGA AGCTCCTCTT 4080
CCTACAGATG ACCAAAATTC TGAGGGAAAT ACGGCTTCCA GTACGAATAG CGATACAAGT 4140
ATGAACAACA ATCAA 4155

(2) INFORMATION FOR SEQ ID NO:2:

(i) SEQUENCE CHARACTERISTICS:
(A) LENGTH: 1385 amino acids
(B) TYPE: amino acid
(C) STRANDEDNESS: single
(D) TOPOLOGY: linear

(ii) MOLECULE TYPE: protein

(iii) HYPOTHETICAL: YES

(iv) ANTI-SENSE: NO

(vi) ORIGINAL SOURCE:
(A) ORGANISM: BACILLUS THURINGIENSIS
(B) STRAIN: PS17
(C) INDIVIDUAL ISOLATE: PS17a

(vii) IMMEDIATE SOURCE:
(B) CLONE: E. coli NM522(pMYC1627) NRRL B-18651

(xi) SEQUENCE DESCRIPTION: SEQ ID NO:2:

Met Ala Ile Leu Asn Glu Leu Tyr Pro Ser Val Pro Tyr Asn Val Leu
1               5                   10                  15

Ala Tyr Thr Pro Pro Ser Phe Leu Pro Asp Ala Gly Thr Gln Ala Thr
            20                  25                  30

Pro Ala Asp Leu Thr Ala Tyr Glu Gln Leu Leu Lys Asn Leu Glu Lys
        35                  40                  45

Gly Ile Asn Ala Gly Thr Tyr Ser Lys Ala Ile Ala Asp Val Leu Lys
    50                  55                  60

Gly Ile Phe Ile Asp Asp Thr Ile Asn Tyr Gln Thr Tyr Val Asn Ile
65                  70                  75                  80

Gly Leu Ser Leu Ile Thr Leu Ala Val Pro Glu Ile Gly Ile Phe Thr
                85                  90                  95

Pro Phe Ile Gly Leu Phe Phe Ala Ala Leu Asn Lys His Asp Ala Pro
            100                 105                 110

Pro Pro Pro Asn Ala Lys Asp Ile Phe Glu Ala Met Lys Pro Ala Ile
        115                 120                 125

Gln Glu Met Ile Asp Arg Thr Leu Thr Ala Asp Glu Gln Thr Phe Leu
    130                 135                 140

Asn Gly Glu Ile Ser Gly Leu Gln Asn Leu Ala Ala Arg Tyr Gln Ser
145                 150                 155                 160
Thr Met Asp Asp Ile Gln Ser His Gly Gly Phe Asn Lys Val Asp Ser
                165                 170                 175
Gly Leu Ile Lys Lys Phe Thr Asp Glu Val Leu Ser Leu Asn Ser Phe
            180                 185                 190
Tyr Thr Asp Arg Leu Pro Val Phe Ile Thr Asp Asn Thr Ala Asp Arg
        195                 200                 205
Thr Leu Leu Gly Leu Pro Tyr Tyr Ala Ile Leu Ala Ser Met His Leu
    210                 215                 220
Met Leu Leu Arg Asp Ile Ile Thr Lys Gly Pro Thr Trp Asp Ser Lys
225                 230                 235                 240
Ile Asn Phe Thr Pro Asp Ala Ile Asp Ser Phe Lys Thr Asp Ile Lys
                245                 250                 255
Asn Asn Ile Lys Leu Tyr Ser Lys Thr Ile Tyr Asp Val Phe Gln Lys
            260                 265                 270
Gly Leu Ala Ser Tyr Gly Thr Pro Ser Asp Leu Glu Ser Phe Ala Lys
        275                 280                 285
Lys Gln Lys Tyr Ile Glu Ile Met Thr Thr His Cys Leu Asp Phe Ala
    290                 295                 300
Arg Leu Phe Pro Thr Phe Asp Pro Asp Leu Tyr Pro Thr Gly Ser Gly
305                 310                 315                 320
Asp Ile Ser Leu Gln Lys Thr Arg Arg Ile Leu Ser Pro Phe Ile Pro
                325                 330                 335
Ile Arg Thr Ala Asp Gly Leu Thr Leu Asn Asn Thr Ser Ile Asp Thr
            340                 345                 350
Ser Asn Trp Pro Asn Tyr Glu Asn Gly Asn Gly Ala Phe Pro Asn Pro
        355                 360                 365
Lys Glu Arg Ile Leu Lys Gln Phe Lys Leu Tyr Pro Ser Trp Arg Ala
    370                 375                 380
Gly Gln Tyr Gly Gly Leu Leu Gln Pro Tyr Leu Trp Ala Ile Glu Val
385                 390                 395                 400
Gln Asp Ser Val Glu Thr Arg Leu Tyr Gly Gln Leu Pro Ala Val Asp
                405                 410                 415
Pro Gln Ala Gly Pro Asn Tyr Val Ser Ile Asp Ser Ser Asn Pro Ile
            420                 425                 430
Ile Gln Ile Asn Met Asp Thr Trp Lys Thr Pro Pro Gln Gly Ala Ser
        435                 440                 445
Gly Trp Asn Thr Asn Leu Met Arg Gly Ser Val Ser Gly Leu Ser Phe
    450                 455                 460
Leu Gln Arg Asp Gly Thr Arg Leu Ser Ala Gly Met Gly Gly Gly Phe
465                 470                 475                 480
Ala Asp Thr Ile Tyr Ser Leu Pro Ala Thr His Tyr Leu Ser Tyr Leu
                485                 490                 495
Tyr Gly Thr Pro Tyr Gln Thr Ser Asp Asn Tyr Ser Gly His Val Gly
            500                 505                 510
Ala Leu Val Gly Val Ser Thr Pro Gln Glu Ala Thr Leu Pro Asn Ile
        515                 520                 525
Ile Gly Gln Pro Asp Glu Gln Gly Asn Val Ser Thr Met Gly Phe Pro
    530                 535                 540
Phe Glu Lys Ala Ser Tyr Gly Gly Thr Val Val Lys Glu Trp Leu Asn
545                 550                 555                 560
Gly Ala Asn Ala Met Lys Leu Ser Pro Gly Gln Ser Ile Gly Ile Pro
                565                 570                 575
Ile Thr Asn Val Thr Ser Gly Glu Tyr Gln Ile Arg Cys Arg Tyr Ala
            580                 585                 590
Ser Asn Asp Asn Thr Asn Val Phe Phe Asn Val Asp Thr Gly Gly Ala
        595                 600                 605

Asn Pro Ile Phe Gln Gln Ile Asn Phe Ala Ser Thr Val Asp Asn Asn
    610                 615                 620

Thr Gly Val Gln Gly Ala Asn Gly Val Tyr Val Val Lys Ser Ile Ala
625                 630                 635                 640

Thr Thr Asp Asn Ser Phe Thr Glu Ile Pro Ala Lys Thr Ile Asn Val
                645                 650                 655

His Leu Thr Asn Gln Gly Ser Ser Asp Val Phe Leu Asp Arg Ile Glu
            660                 665                 670

Phe Ile Pro Phe Ser Leu Pro Leu Ile Tyr His Gly Ser Tyr Asn Thr
        675                 680                 685

Ser Ser Gly Ala Asp Asp Val Leu Trp Ser Ser Ser Asn Met Asn Tyr
    690                 695                 700

Tyr Asp Ile Ile Val Asn Gly Gln Ala Asn Ser Ser Ser Ile Ala Ser
705                 710                 715                 720

Ser Met His Leu Leu Asn Lys Gly Lys Val Ile Lys Thr Ile Asp Ile
                725                 730                 735

Pro Gly His Ser Glu Thr Phe Phe Ala Thr Phe Pro Val Pro Glu Gly
            740                 745                 750

Phe Asn Glu Val Arg Ile Leu Ala Gly Leu Pro Glu Val Ser Gly Asn
        755                 760                 765

Ile Thr Val Gln Ser Asn Asn Pro Pro Gln Pro Ser Asn Asn Gly Gly
    770                 775                 780

Gly Asp Gly Gly Gly Asn Gly Gly Gly Asp Gly Gly Gln Tyr Asn Phe
785                 790                 795                 800

Ser Leu Ser Gly Ser Asp His Thr Thr Ile Tyr His Gly Lys Leu Glu
                805                 810                 815

Thr Gly Ile His Val Gln Gly Asn Tyr Thr Tyr Thr Gly Thr Pro Val
            820                 825                 830

Leu Ile Leu Asn Ala Tyr Arg Asn Asn Thr Val Val Ser Ser Ile Pro
        835                 840                 845

Val Tyr Ser Pro Phe Asp Ile Thr Ile Gln Thr Glu Ala Asp Ser Leu
    850                 855                 860

Glu Leu Glu Leu Gln Pro Arg Tyr Gly Phe Ala Thr Val Asn Gly Thr
865                 870                 875                 880

Ala Thr Val Lys Ser Pro Asn Val Asn Tyr Asp Arg Ser Phe Lys Leu
                885                 890                 895

Pro Ile Asp Leu Gln Asn Ile Thr Thr Gln Val Asn Ala Leu Phe Ala
            900                 905                 910

Ser Gly Thr Gln Asn Met Leu Ala His Asn Val Ser Asp His Asp Ile
        915                 920                 925

Glu Glu Val Val Leu Lys Val Asp Ala Leu Ser Asp Glu Val Phe Gly
    930                 935                 940

Asp Glu Lys Lys Ala Leu Arg Lys Leu Val Asn Gln Ala Lys Arg Leu
945                 950                 955                 960

Ser Arg Ala Arg Asn Leu Leu Ile Gly Gly Ser Phe Glu Asn Trp Asp
                965                 970                 975

Ala Trp Tyr Lys Gly Arg Asn Val Val Thr Val Ser Asp His Glu Leu
            980                 985                 990

Phe Lys Ser Asp His Val Leu Leu Pro Pro Pro Gly Leu Ser Pro Ser
        995                 1000                1005

Tyr Ile Phe Gln Lys Val Glu Glu Ser Lys Leu Lys Pro Asn Thr Arg
    1010                1015                1020

Tyr Ile Val Ser Gly Phe Ile Ala His Gly Lys Asp Leu Glu Ile Val
1025                1030                1035                1040

Val Ser Arg Tyr Gly Gln Glu Val Gln Lys Val Val Gln Val Pro Tyr
                1045                1050                1055

Gly Glu Ala Phe Pro Leu Thr Ser Asn Gly Pro Val Cys Cys Pro Pro
            1060                1065                1070

Arg Ser Thr Ser Asn Gly Thr Leu Gly Asp Pro His Phe Phe Ser Tyr
        1075                1080                1085

Ser Ile Asp Val Gly Ala Leu Asp Leu Gln Ala Asn Pro Gly Ile Glu
    1090                1095                1100

Phe Gly Leu Arg Ile Val Asn Pro Thr Gly Met Ala Arg Val Ser Asn
1105                1110                1115                1120

Leu Glu Ile Arg Glu Asp Arg Pro Leu Ala Ala Asn Glu Ile Arg Gln
                1125                1130                1135

Val Gln Arg Val Ala Arg Asn Trp Arg Thr Glu Tyr Glu Lys Glu Arg
            1140                1145                1150

Ala Glu Val Thr Ser Leu Ile Gln Pro Val Ile Asn Arg Ile Asn Gly
        1155                1160                1165

Leu Tyr Glu Asn Gly Asn Trp Asn Gly Ser Ile Arg Ser Asp Ile Ser
    1170                1175                1180

Tyr Gln Asn Ile Asp Ala Ile Val Leu Pro Thr Leu Pro Lys Leu Arg
1185                1190                1195                1200

His Trp Phe Met Ser Asp Arg Phe Ser Glu Gln Gly Asp Ile Met Ala
                1205                1210                1215

Lys Phe Gln Gly Ala Leu Asn Arg Ala Tyr Ala Gln Leu Glu Gln Ser
            1220                1225                1230

Thr Leu Leu His Asn Gly His Phe Thr Lys Asp Ala Ala Asn Trp Thr
        1235                1240                1245

Ile Glu Gly Asp Ala His Gln Ile Thr Leu Glu Asp Gly Arg Arg Val
    1250                1255                1260

Leu Arg Leu Pro Asp Trp Ser Ser Ser Val Ser Gln Met Ile Glu Ile
1265                1270                1275                1280

Glu Asn Phe Asn Pro Asp Lys Glu Tyr Asn Leu Val Phe His Gly Gln
                1285                1290                1295

Gly Glu Gly Thr Val Thr Leu Glu His Gly Glu Glu Thr Lys Tyr Ile
            1300                1305                1310

Glu Thr His Thr His His Phe Ala Asn Phe Thr Thr Ser Gln Arg Gln
        1315                1320                1325

Gly Leu Thr Phe Glu Ser Asn Lys Val Thr Val Thr Ile Ser Ser Glu
    1330                1335                1340

Asp Gly Glu Phe Leu Val Asp Asn Ile Ala Leu Val Glu Ala Pro Leu
1345                1350                1355                1360

Pro Thr Asp Asp Gln Asn Ser Glu Gly Asn Thr Ala Ser Ser Thr Asn
                1365                1370                1375

Ser Asp Thr Ser Met Asn Asn Asn Gln
            1380                1385

(2) INFORMATION FOR SEQ ID NO:3:

(i) SEQUENCE CHARACTERISTICS:
(A) LENGTH: 3867 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Bacillus thuringiensis
(B) STRAIN: PS17
(C) INDIVIDUAL ISOLATE: PS17b

(vii) IMMEDIATE SOURCE:
(B) CLONE: E. coli NM522(pMYC1628) NRRL B-18652

(xi) SEQUENCE DESCRIPTION: SEQ ID NO:3:

ATGGCAATTT TAAATGAATT ATATCCATCT GTACCTTATA ATGTATTGGC GTATACGCCA 60

CCCTCTTTTT TACCTGATGC GGGTACACAA GCTACACCTG CTGACTTAAC AGCTTATGAA 120
CAATTGTTGA AAAATTTAGA AAAAGGGATA AATGCTGGAA CTTATTCGAA AGCAATAGCT 180
GATGTACTTA AAGGTATTTT TATAGATGAT ACAATAAATT ATCAAACATA TGTAAATATT 240
GGTTTAAGTT TAATTACATT AGCTGTACCG GAAATTGGTA TTTTTACACC TTTCATCGGT 300
TTGTTTTTTG CTGCATTGAA TAAACATGAT GCTCCACCTC CTCCTAATGC AAAAGATATA 360
TTTGAGGCTA TGAAACCAGC GATTCAAGAG ATGATTGATA GAACTTTAAC TGCGGATGAG 420
CAAACATTTT TAAATGGGGA AATAAGTGGT TTACAAAATT TAGCAGCAAG ATACCAGTCT 480
ACAATGGATG ATATTCAAAG CCATGGAGGA TTTAATAAGG TAGATTCTGG ATTAATTAAA 540
AAGTTTACAG ATGAGGTACT ATCTTTAAAT AGTTTTTATA CAGATCGTTT ACCTGTATTT 600
ATTACAGATA ATACAGCGGA TCGAACTTTG TTAGGTCTTC CTTATTATGC TATACTTGCG 660
AGCATGCATC TTATGTTATT AAGAGATATC ATTACTAAGG GTCCGACATG GGATTCTAAA 720
ATTAATTTCA CACCAGATGC AATTGATTCC TTTAAAACCG ATATTAAAAA TAATATAAAG 780
CTTTACTCTA AAACTATTTA TGACGTATTT CAGAAGGGAC TTGCTTCATA CGGAACGCCT 840
TCTGATTTAG AGTCCTTTGC AAAAAAACAA AAATATATTG AAATTATGAC AACACATTGT 900
TTAGATTTTG CAAGATTGTT TCCTACTTTT GATCCAGATC TTTATCCAAC AGGATCAGGT 960
GATATAAGTT TACAAAAAAC ACGTAGAATT CTTTCTCCTT TTATCCCTAT ACGTACTGCA 1020
GATGGGTTAA CATTAAATAA TACTTCAATT GATACTTCAA ATTGGCCTAA TTATGAAAAT 1080
GGGAATGGCG CGTTTCCAAA CCCAAAAGAA AGAATATTAA AACAATTCAA ACTGTATCCT 1140
AGTTGGAGAG CGGCACAGTA CGGTGGGCTT TTACAACCTT ATTTATGGGC AATAGAAGTC 1200
CAAGATTCTG TAGAGACTCG TTTGTATGGG CAGCTTCCAG CTGTAGATCC ACAGGCAGGG 1260
CCTAATTATG TTTCCATAGA TTCTTCTAAT CCAATCATAC AAATAAATAT GGATACTTGG 1320
AAAACACCAC CACAAGGTGC GAGTGGGTGG AATACAAATT TAATGAGAGG AAGTGTAAGC 1380
GGGTTAAGTT TTTTACAACG AGATGGTACG AGACTTAGTG CTGGTATGGG TGGTGGTTTT 1440
GCTGATACAA TATATAGTCT CCCTGCAACT CATTATCTTT CTTATCTCTA TGGAACTCCT 1500
TATCAAACTT CTGATAACTA TTCTGGTCAC GTTGGTGCAT TGGTAGGTGT GAGTACGCCT 1560
CAAGAGGCTA CTCTTCCTAA TATTATAGGT CAACCAGATG AACAGGGAAA TGTATCTACA 1620
ATGGGATTTC CGTTTGAAAA AGCTTCTTAT GGAGGTACAG TTGTTAAAGA ATGGTTAAAT 1680
GGTGCGAATG CGATGAAGCT TTCTCCTGGG CAATCTATAG GTATTCCTAT TACAAATGTA 1740
ACAAGTGGAG AATATCAAAT TCGTTGTCGT TATGCAAGTA ATGATAATAC TAACGTTTTC 1800
TTTAATGTAG ATACTGGTGG AGCAAATCCA ATTTTCCAAC AGATAAACTT TGCATCTACT 1860
GTAGATAATA ATACGGGAGT ACAAGGAGCA AATGGTGTCT ATGTAGTCAA ATCTATTGCT 1920
ACAACTGATA ATTCTTTTAC AGTAAAAATT CCTGCGAAGA CGATTAATGT TCATTTAACC 1980
AACCAAGGTT CTTCTGATGT CTTTTTAGAT CGTATTGAGT TTGTTCCAAT TCTAGAATCA 2040
AATACTGTAA CTATATTCAA CAATTCATAT ACTACAGGTT CAGCAAATCT TATACCAGCA 2100
ATAGCTCCTC TTTGGAGTAC TAGTTCAGAT AAAGCCCTTA CAGGTTCTAT GTCAATAACA 2160
GGTCGAACTA CCCCTAACAG TGATGATGCT TTGCTTCGAT TTTTTAAAAC TAATTATGAT 2220
ACACAAACCA TTCCTATTCC GGGTTCCGGA AAAGATTTTA CAAATACTCT AGAAATACAA 2280
GACATAGTTT CTATTGATAT TTTTGTCGGA TCTGGTCTAC ATGGATCCGA TGGATCTATA 2340
AAATTAGATT TTACCAATAA TAATAGTGGT AGTGGTGGCT CTCCAAAGAG TTTCACCGAG 2400
CAAAATGATT TAGAGAATAT CACAACACAA GTGAATGCTC TATTCACATC TAATACACAA 2460
GATGCACTTG CAACAGATGT GAGTGATCAT GATATTGAAG AAGTGGTTCT AAAAGTAGAT 2520
GCATTATCTG ATGAAGTGTT TGGAAAAGAG AAAAAAACAT TGCGTAAATT TGTAAATCAA 2580
GCGAAGCGCT TAAGCAAGGC GCGTAATCTC CTGGTAGGAG GCAATTTTGA TAACTTGGAT 2640
GCTTGGTATA GAGGAAGAAA TGTAGTAAAC GTATCTAATC ACGAACTGTT GAAGAGTGAT 2700

CATGTATTAT TACCACCACC AGGATTGTCT CCATCTTATA TTTTCCAAAA AGTGGAGGAA 2760
TCTAAATTAA AACGAAATAC ACGTTATACG GTTTCTGGAT TTATTGCGCA TGCAACAGAT 2820
TTAGAAATTG TGGTTTCTCG TTATGGGCAA GAAATAAAGA AAGTGGTGCA AGTTCCTTAT 2880
GGAGAAGCAT TCCCATTAAC ATCAAGTGGA CCAGTTTGTT GTATCCCACA TTCTACAAGT 2940
AATGGAACTT TAGGCAATCC ACATTTCTTT AGTTACAGTA TTGATGTAGG TGCATTAGAT 3000
GTAGACACAA ACCCTGGTAT TGAATTCGGT CTTCGTATTG TAAATCCAAC TGGAATGGCA 3060
CGCGTAAGCA ATTTGGAAAT TCGTGAAGAT CGTCCATTAG CAGCAAATGA AATACGACAA 3120
GTACAACGTG TCGCAAGAAA TTGGAGAACC GAGTATGAGA AAGAACGTGC GGAAGTAACA 3180
AGTTTAATTC AACCTGTTAT CAATCGAATC AATGGATTGT ATGACAATGG AAATTGGAAC 3240
GGTTCTATTC GTTCAGATAT TTCGTATCAG AATATAGACG CGATTGTATT ACCAACGTTA 3300
CCAAAGTTAC GCCATTGGTT TATGTCAGAT AGATTTAGTG AACAAGGAGA TATCATGGCT 3360
AAATTCCAAG GTGCATTAAA TCGTGCGTAT GCACAACTGG AACAAAATAC GCTTCTGCAT 3420
AATGGTCATT TTACAAAAGA TGCAGCCAAT TGGACGGTAG AAGGCGATGC ACATCAGGTA 3480
GTATTAGAAG ATGGTAAACG TGTATTACGA TTGCCAGATT GGTCTTCGAG TGTGTCTCAA 3540
ACGATTGAAA TCGAGAATTT TGATCCAGAT AAAGAATATC AATTAGTATT TCATGGGCAA 3600
GGAGAAGGAA CGGTTACGTT GGAGCATGGA GAAGAAACAA AATATATAGA AACGCATACA 3660
CATCATTTTG CGAATTTTAC AACTTCTCAA CGTCAAGGAC TCACGTTTGA ATCAAATAAA 3720
GTGACAGTGA CCATTTCTTC AGAAGATGGA GAATTCTTAG TGGATAATAT TGCGCTTGTG 3780
GAAGCTCCTC TTCCTACAGA TGACCAAAAT TCTGAGGGAA ATACGGCTTC CAGTACGAAT 3840
AGCGATACAA GTATGAACAA CAATCAA 3867

(2) INFORMATION FOR SEQ ID NO:4:

(i) SEQUENCE CHARACTERISTICS:
(A) LENGTH: 1289 amino acids
(B) TYPE: amino acid
(C) STRANDEDNESS: single
(D) TOPOLOGY: linear

(ii) MOLECULE TYPE: protein

(iii) HYPOTHETICAL: YES

(iv) ANTI-SENSE: NO

(vi) ORIGINAL SOURCE:
(A) ORGANISM: BACILLUS THURINGIENSIS
(B) STRAIN: PS17
(C) INDIVIDUAL ISOLATE: PS17b

(vii) IMMEDIATE SOURCE:
(B) CLONE: E. coli NM522(pMYC1628) NRRL B-18652

(xi) SEQUENCE DESCRIPTION: SEQ ID NO:4:

Met Ala Ile Leu Asn Glu Leu Tyr Pro Ser Val Pro Tyr Asn Val Leu
1               5                   10                  15

Ala Tyr Thr Pro Pro Ser Phe Leu Pro Asp Ala Gly Thr Gln Ala Thr
            20                  25                  30

Pro Ala Asp Leu Thr Ala Tyr Glu Gln Leu Leu Lys Asn Leu Glu Lys
        35                  40                  45

Gly Ile Asn Ala Gly Thr Tyr Ser Lys Ala Ile Ala Asp Val Leu Lys
    50                  55                  60

Gly Ile Phe Ile Asp Asp Thr Ile Asn Tyr Gln Thr Tyr Val Asn Ile
65                  70                  75                  80

Gly Leu Ser Leu Ile Thr Leu Ala Val Pro Glu Ile Gly Ile Phe Thr
                85                  90                  95

Pro Phe Ile Gly Leu Phe Phe Ala Ala Leu Asn Lys His Asp Ala Pro
            100                 105                 110

Pro Pro Pro Asn Ala Lys Asp Ile Phe Glu Ala Met Lys Pro Ala Ile
        115                 120                 125

Gln Glu Met Ile Asp Arg Thr Leu Thr Ala Asp Glu Gln Thr Phe Leu
    130                 135                 140
Asn Gly Glu Ile Ser Gly Leu Gln Asn Leu Ala Ala Arg Tyr Gln Ser
145                 150                 155                 160
Thr Met Asp Asp Ile Gln Ser His Gly Gly Phe Asn Lys Val Asp Ser
                165                 170                 175
Gly Leu Ile Lys Lys Phe Thr Asp Glu Val Leu Ser Leu Asn Ser Phe
            180                 185                 190
Tyr Thr Asp Arg Leu Pro Val Phe Ile Thr Asp Asn Thr Ala Asp Arg
        195                 200                 205
Thr Leu Leu Gly Leu Pro Tyr Tyr Ala Ile Leu Ala Ser Met His Leu
    210                 215                 220
Met Leu Leu Arg Asp Ile Ile Thr Lys Gly Pro Thr Trp Asp Ser Lys
225                 230                 235                 240
Ile Asn Phe Thr Pro Asp Ala Ile Asp Ser Phe Lys Thr Asp Ile Lys
                245                 250                 255
Asn Asn Ile Lys Leu Tyr Ser Lys Thr Ile Tyr Asp Val Phe Gln Lys
            260                 265                 270
Gly Leu Ala Ser Tyr Gly Thr Pro Ser Asp Leu Glu Ser Phe Ala Lys
        275                 280                 285
Lys Gln Lys Tyr Ile Glu Ile Met Thr Thr His Cys Leu Asp Phe Ala
    290                 295                 300
Arg Leu Phe Pro Thr Phe Asp Pro Asp Leu Tyr Pro Thr Gly Ser Gly
305                 310                 315                 320
Asp Ile Ser Leu Gln Lys Thr Arg Arg Ile Leu Ser Pro Phe Ile Pro
                325                 330                 335
Ile Arg Thr Ala Asp Gly Leu Thr Leu Asn Asn Thr Ser Ile Asp Thr
            340                 345                 350
Ser Asn Trp Pro Asn Tyr Glu Asn Gly Asn Gly Ala Phe Pro Asn Pro
        355                 360                 365
Lys Glu Arg Ile Leu Lys Gln Phe Lys Leu Tyr Pro Ser Trp Arg Ala
    370                 375                 380
Ala Gln Tyr Gly Gly Leu Leu Gln Pro Tyr Leu Trp Ala Ile Glu Val
385                 390                 395                 400
Gln Asp Ser Val Glu Thr Arg Leu Tyr Gly Gln Leu Pro Ala Val Asp
                405                 410                 415
Pro Gln Ala Gly Pro Asn Tyr Val Ser Ile Asp Ser Ser Asn Pro Ile
            420                 425                 430
Ile Gln Ile Asn Met Asp Thr Trp Lys Thr Pro Pro Gln Gly Ala Ser
        435                 440                 445
Gly Trp Asn Thr Asn Leu Met Arg Gly Ser Val Ser Gly Leu Ser Phe
    450                 455                 460
Leu Gln Arg Asp Gly Thr Arg Leu Ser Ala Gly Met Gly Gly Gly Phe
465                 470                 475                 480
Ala Asp Thr Ile Tyr Ser Leu Pro Ala Thr His Tyr Leu Ser Tyr Leu
                485                 490                 495
Tyr Gly Thr Pro Tyr Gln Thr Ser Asp Asn Tyr Ser Gly His Val Gly
            500                 505                 510
Ala Leu Val Gly Val Ser Thr Pro Gln Glu Ala Thr Leu Pro Asn Ile
        515                 520                 525
Ile Gly Gln Pro Asp Glu Gln Gly Asn Val Ser Thr Met Gly Phe Pro
    530                 535                 540
Phe Glu Lys Ala Ser Tyr Gly Gly Thr Val Val Lys Glu Trp Leu Asn
545                 550                 555                 560
Gly Ala Asn Ala Met Lys Leu Ser Pro Gly Gln Ser Ile Gly Ile Pro
                565                 570                 575
Ile Thr Asn Val Thr Ser Gly Glu Tyr Gln Ile Arg Cys Arg Tyr Ala
            580                 585                 590

Ser Asn Asp Asn Thr Asn Val Phe Phe Asn Val Asp Thr Gly Gly Ala
        595                 600                 605
Asn Pro Ile Phe Gln Gln Ile Asn Phe Ala Ser Thr Val Asp Asn Asn
    610                 615                 620
Thr Gly Val Gln Gly Ala Asn Gly Val Tyr Val Val Lys Ser Ile Ala
625                 630                 635                 640
Thr Thr Asp Asn Ser Phe Thr Val Lys Ile Pro Ala Lys Thr Ile Asn
                645                 650                 655
Val His Leu Thr Asn Gln Gly Ser Ser Asp Val Phe Leu Asp Arg Ile
            660                 665                 670
Glu Phe Val Pro Ile Leu Glu Ser Asn Thr Val Thr Ile Phe Asn Asn
        675                 680                 685
Ser Tyr Thr Thr Gly Ser Ala Asn Leu Ile Pro Ala Ile Ala Pro Leu
    690                 695                 700
Trp Ser Thr Ser Ser Asp Lys Ala Leu Thr Gly Ser Met Ser Ile Thr
705                 710                 715                 720
Gly Arg Thr Thr Pro Asn Ser Asp Asp Ala Leu Leu Arg Phe Phe Lys
                725                 730                 735
Thr Asn Tyr Asp Thr Gln Thr Ile Pro Ile Pro Gly Ser Gly Lys Asp
            740                 745                 750
Phe Thr Asn Thr Leu Glu Ile Gln Asp Ile Val Ser Ile Asp Ile Phe
        755                 760                 765
Val Gly Ser Gly Leu His Gly Ser Asp Gly Ser Ile Lys Leu Asp Phe
    770                 775                 780
Thr Asn Asn Asn Ser Gly Ser Gly Gly Ser Pro Lys Ser Phe Thr Glu
785                 790                 795                 800
Gln Asn Asp Leu Glu Asn Ile Thr Thr Gln Val Asn Ala Leu Phe Thr
                805                 810                 815
Ser Asn Thr Gln Asp Ala Leu Ala Thr Asp Val Ser Asp His Asp Ile
            820                 825                 830
Glu Glu Val Val Leu Lys Val Asp Ala Leu Ser Asp Glu Val Phe Gly
        835                 840                 845
Lys Glu Lys Lys Thr Leu Arg Lys Phe Val Asn Gln Ala Lys Arg Leu
    850                 855                 860
Ser Lys Ala Arg Asn Leu Leu Val Gly Gly Asn Phe Asp Asn Leu Asp
865                 870                 875                 880
Ala Trp Tyr Arg Gly Arg Asn Val Val Asn Val Ser Asn His Glu Leu
                885                 890                 895
Leu Lys Ser Asp His Val Leu Leu Pro Pro Pro Gly Leu Ser Pro Ser
            900                 905                 910
Tyr Ile Phe Gln Lys Val Glu Glu Ser Lys Leu Lys Arg Asn Thr Arg
        915                 920                 925
Tyr Thr Val Ser Gly Phe Ile Ala His Ala Thr Asp Leu Glu Ile Val
    930                 935                 940
Val Ser Arg Tyr Gly Gln Glu Ile Lys Lys Val Val Gln Val Pro Tyr
945                 950                 955                 960
Gly Glu Ala Phe Pro Leu Thr Ser Ser Gly Pro Val Cys Cys Ile Pro
                965                 970                 975
His Ser Thr Ser Asn Gly Thr Leu Gly Asn Pro His Phe Phe Ser Tyr
            980                 985                 990
Ser Ile Asp Val Gly Ala Leu Asp Val Asp Thr Asn Pro Gly Ile Glu
        995                 1000                1005
Phe Gly Leu Arg Ile Val Asn Pro Thr Gly Met Ala Arg Val Ser Asn
    1010                1015                1020
Leu Glu Ile Arg Glu Asp Arg Pro Leu Ala Ala Asn Glu Ile Arg Gln
1025                1030                1035                1040
Val Gln Arg Val Ala Arg Asn Trp Arg Thr Glu Tyr Glu Lys Glu Arg
                1045                1050                1055

Ala Glu Val Thr Ser Leu Ile Gln Pro Val Ile Asn Arg Ile Asn Gly
            1060                1065                1070
Leu Tyr Asp Asn Gly Asn Trp Asn Gly Ser Ile Arg Ser Asp Ile Ser
        1075                1080                1085
Tyr Gln Asn Ile Asp Ala Ile Val Leu Pro Thr Leu Pro Lys Leu Arg
    1090                1095                1100
His Trp Phe Met Ser Asp Arg Phe Ser Glu Gln Gly Asp Ile Met Ala
1105                1110                1115                1120
Lys Phe Gln Gly Ala Leu Asn Arg Ala Tyr Ala Gln Leu Glu Gln Asn
                1125                1130                1135
Thr Leu Leu His Asn Gly His Phe Thr Lys Asp Ala Ala Asn Trp Thr
            1140                1145                1150
Val Glu Gly Asp Ala His Gln Val Val Leu Glu Asp Gly Lys Arg Val
        1155                1160                1165
Leu Arg Leu Pro Asp Trp Ser Ser Ser Val Ser Gln Thr Ile Glu Ile
    1170                1175                1180
Glu Asn Phe Asp Pro Asp Lys Glu Tyr Gln Leu Val Phe His Gly Gln
1185                1190                1195                1200
Gly Glu Gly Thr Val Thr Leu Glu His Gly Glu Glu Thr Lys Tyr Ile
                1205                1210                1215
Glu Thr His Thr His His Phe Ala Asn Phe Thr Thr Ser Gln Arg Gln
            1220                1225                1230
Gly Leu Thr Phe Glu Ser Asn Lys Val Thr Val Thr Ile Ser Ser Glu
        1235                1240                1245
Asp Gly Glu Phe Leu Val Asp Asn Ile Ala Leu Val Glu Ala Pro Leu
    1250                1255                1260
Pro Thr Asp Asp Gln Asn Ser Glu Gly Asn Thr Ala Ser Ser Thr Asn
1265                1270                1275                1280
Ser Asp Thr Ser Met Asn Asn Asn Gln
                1285

(2) INFORMATION FOR SEQ ID NO:5:

    (i) SEQUENCE CHARACTERISTICS:
        (A) LENGTH: 3771 base pairs
        (B) TYPE: nucleic acid
        (C) STRANDEDNESS: double
        (D) TOPOLOGY: linear

   (ii) MOLECULE TYPE: DNA (genomic)

  (iii) HYPOTHETICAL: NO

   (iv) ANTI-SENSE: NO

   (vi) ORIGINAL SOURCE:
        (A) ORGANISM: Bacillus thuringiensis
        (C) INDIVIDUAL ISOLATE: 33F2

  (vii) IMMEDIATE SOURCE:
        (B) CLONE: E. coli NM522(pMYC2316) B-18785

   (ix) FEATURE:
        (A) NAME/KEY: misc_feature
        (B) LOCATION: 4..24
        (D) OTHER INFORMATION: /function= "oligonucleotide
              hybridization probe"
              /product= "GCA/T ACA/T TTA AAT GAA GTA/T TAT"
              /standard_name= "probe a"
              /note= "Probe A"

   (ix) FEATURE:
        (A) NAME/KEY: misc_feature
        (B) LOCATION: 13..33
        (D) OTHER INFORMATION: /function= "oligonucleotide
              hybridization probe"
              /product= "AAT GAA GTA/T TAT CCA/T GTA/T AAT"
              /standard_name= "Probe B"
              /label= probe-b
              /note= "probe b"

   (xi) SEQUENCE DESCRIPTION: SEQ ID NO:5:

ATGGCTACAC TTAATGAAGT ATATCCTGTG AATTATAATG TATTATCTTC TGATGCTTTT 60
CAACAATTAG ATACAACAGG TTTTAAAAGT AAATATGATG AAATGATAAA AGCATTCGAA 120
AAAAAATGGA AAAAAGGGGC AAAAGGAAAA GACCTTTTAG ATGTTGCATG GACTTATATA 180
ACTACAGGAG AAATTGACCC TTTAAATGTA ATTAAAGGTG TTTTATCTGTATTAACTTTA 240
ATTCCTGAAG TTGGTACTGT GGCCTCTGCA GCAAGTACTA TTGTAAGTTT TATTTGGCCT 300
AAAATATTTG GAGATAAACC AAATGCAAAA AATATATTTG AAGAGCTCAA GCCTCAAATT 360
GAAGCATTAA TTCAACAAGA TATAACAAAC TATCAAGATG CAATTAATCA AAAAAAATTT 420
GACAGTCTTC AGAAAACAAT TAATCTATAT ACAGTAGCTA TAGATAACAA TGATTACGTA 480
ACAGCAAAAA CGCAACTCGA AAATCTAAAT TCTATACTTA CCTCAGATAT CTCCATATTT 540
ATTCCAGAAG GATATGAAAC TGGAGGTTTA CCTTATTATG CTATGGTTGC TAATGCTCAT 600
ATATTATTGT TAAGAGACGC TATAGTTAAT GCAGAGAAAT TAGGCTTTAG TGATAAAGAA 660
GTAGACACAC ATAAAAAATA TATCAAAATG ACAATACACA ATCATACTGA AGCAGTAATA 720
AAAGCATTCT TAAATGGACT TGACAAATTT AAGAGTTTAG ATGTAAATAG CTATAATAAA 780
AAAGCAAATT ATATTAAAGG TATGACAGAA ATGGTTCTTG ATCTAGTTGC TCTATGGCCA 840
ACTTTCGATC CAGATCATTA TCAAAAAGAA GTAGAAATTG AATTTACAAG AACTATTTCT 900
TCTCCAATTT ACCAACCTGT ACCTAAAAAC ATGCAAAATA CCTCTAGCTC TATTGTACCT 960
AGCGATCTAT TTCACTATCA AGGAGATCTT GTAAAATTAG AATTTTCTAC AAGAACGGAC 1020
AACGATGGTC TTGCAAAAAT TTTTACTGGT ATTCGAAACA CATTCTACAA ATCGCCTAAT 1080
ACTCATGAAA CATACCATGT AGATTTTAGT TATAATACCC AATCTAGTGG TAATATTTCA 1140
AGAGGCTCTT CAAATCCGAT TCCAATTGAT CTTAATAATC CCATTATTTC AACTTGTATT 1200
AGAAATTCAT TTTATAAGGC AATAGCGGGA TCTTCTGTTT TAGTTAATTT TAAAGATGGC 1260
ACTCAAGGGT ATGCATTTGC CCAAGCACCA ACAGGAGGTG CCTGGGACCA TTCTTTTATT 1320
GAATCTGATG GTGCCCCAGA AGGGCATAAA TTAAACTATA TTTATACTTC TCCAGGTGAT 1380
ACATTAAGAG ATTTCATCAA TGTATATACT CTTATAAGTA CTCCAACTAT AAATGAACTA 1440
TCAACAGAAA AAATCAAAGG CTTTCCTGCG GAAAAAGGAT ATATCAAAAA TCAAGGGATC 1500
ATGAAATATT ACGGTAAACC AGAATATATT AATGGAGCTC AACCAGTTAA TCTGGAAAAC 1560
CAGCAAACAT TAATATTCGA ATTTCATGCT TCAAAAACAG CTCAATATAC CATTCGTATA 1620
CGTTATGCCA GTACCCAAGG AACAAAAGGT TATTTTCGTT TAGATAATCA GGAACTGCAA 1680
ACGCTTAATA TACCTACTTC ACACAACGGT TATGTAACCG GTAATATTGG TGAAAATTAT 1740
GATTTATATA CAATAGGTTC ATATACAATT ACAGAAGGTA ACCATACTCT TCAAATCCAA 1800
CATAATGATA AAAATGGAAT GGTTTTAGAT CGTATTGAAT TTGTTCCTAA AGATTCACTT 1860
CAAGATTCAC CTCAAGATTC ACCTCCAGAA GTTCACGAAT CAACAATTAT TTTTGATAAA 1920
TCATCTCCAA CTATATGGTC TTCTAACAAA CACTCATATA GCCATATACA TTTAGAAGGA 1980
TCATATACAA GTCAGGGAAG TTATCCACAC AATTTATTAA TTAATTTATT TCATCCTACA 2040
GACCCTAACA GAAATCATAC TATTCATGTT AACAATGGTG ATATGAATGT TGATTATGGA 2100
AAAGATTCTG TAGCCGATGG GTTAAATTTT AATAAAATAA CTGCTACGAT ACCAAGTGAT 2160
GCTTGGTATA GCGGTACTAT TACTTCTATG CACTTATTTA ATGATAATAA TTTTAAAACA 2220
ATAACTCCTA AATTTGAACT TTCTAATGAA TTAGAAAACA TCACAACTCA AGTAAATGCT 2280
TTATTCGCAT CTAGTGCACA AGATACTCTC GCAAGTAATG TAAGTGATTA CTGGATTGAA 2340
CAGGTCGTTA TGAAAGTCGA TGCCTTATCA GATGAAGTAT TTGGAAAAGA GAAAAAAGCA 2400
TTACGTAAAT TGGTAAATCA AGCAAAACGT CTCAGTAAAA TACGAAATCT TCTCATAGGT 2460
GGTAATTTTG ACAATTTAGT CGCTTGGTAT ATGGGAAAAG ATGTAGTAAA AGAATCGGAT 2520
CATGAATTAT TTAAAAGTGA TCATGTCTTA CTACCTCCCC CAACATTCCA TCCTTCTTAT 2580
ATTTTCCAAA AGGTGGAAGA ATCAAAACTA AAACCAAATA CACGTTATAC TATTTCTGGT 2640

TTTATCGCAC ATGGAGAAGA TGTAGAGCTT GTTGTCTCTC GTTATGGGCA AGAAATACAA 2700
AAAGTGATGC AAGTGCCATA TGAAGAAGCA CTTCCTCTTA CATCTGAATC TAATTCTAGT 2760
TGTTGTGTTC CAAATTTAAA TATAAATGAA ACACTAGCTG ATCCACATTT CTTTAGTTAT 2820
AGCATCGATG TTGGTTCTCT GGAAATGGAA GCGAATCCTG GTATTGAATT TGGTCTCCGT 2880
ATTGTCAAAC CAACAGGTAT GGCACGTGTA AGTAATTTAG AAATTCGAGA AGACCGTCCA 2940
TTAACAGCAA AAGAAATTCG TCAAGTACAA CGTGCAGCAA GAGATTGGAA ACAAAACTAT 3000
GAACAAGAAC GAACAGAGAT CACAGCTATA ATTCAACCTG TTCTTAATCA AATTAATGCG 3060
TTATACGAAA ATGAAGATTG GAATGGTTCT ATTCGTTCAA ATGTTTCCTA TCATGATCTA 3120
GAGCAAATTA TGCTTCCTAC TTTATTAAAA ACTGAGGAAA TAAATTGTAA TTATGATCAT 3180
CCAGCTTTTT TATTAAAAGT ATATCATTGG TTTATGACAG ATCGTATAGG AGAACATGGT 3240
ACTATTTTAG CACGTTTCCA AGAAGCATTA GATCGTGCAT ATACACAATT AGAAAGTCGT 3300
AATCTCCTGC ATAACGGTCA TTTTACAACT GATACAGCGA ATTGGACAAT AGAAGGAGAT 3360
GCCCATCATA CAATCTTAGA AGATGGTAGA CGTGTGTTAC GTTTACCAGA TTGGTCTTCT 3420
AATGCAACTC AAACAATTGA AATTGAAGAT TTTGACTTAG ATCAAGAATA CCAATTGCTC 3480
ATTCATGCAA AAGGAAAAGG TTCCATTACT TTACAACATG GAGAAGAAAA CGAATATGTG 3540
GAAACACATA CTCATCATAC AAATGATTTT ATAACATCCC AAAATATTCC TTTCACTTTT 3600
AAAGGAAATC AAATTGAAGT CCATATTACT TCAGAAGATG GAGAGTTTTT AATCGATCAC 3660
ATTACAGTAA TAGAAGTTTC TAAAACAGAC ACAAATACAA ATATTATTGA AAATTCACCA 3720
ATCAATACAA GTATGAATAG TAATGTAAGA GTAGATATAC CAAGAAGTCT C 3771

(2) INFORMATION FOR SEQ ID NO:6:

(i) SEQUENCE CHARACTERISTICS:
(A) LENGTH: 1257 amino acids
(B) TYPE: amino acid
(C) STRANDEDNESS: single
(D) TOPOLOGY: linear

(ii) MOLECULE TYPE: protein

(iii) HYPOTHETICAL: YES

(iv) ANTI-SENSE: NO

(vi) ORIGINAL SOURCE:
(A) ORGANISM: Bacillus thuringiensis
(C) INDIVIDUAL ISOLATE: PS33F2

(vii) IMMEDIATE SOURCE:
(B) CLONE: E. coli NM522(pMYC2316) B-18785

(ix) FEATURE:
(A) NAME/KEY: Protein
(B) LOCATION: 1..1257

(xi) SEQUENCE DESCRIPTION: SEQ ID NO:6:

Met Ala Thr Leu Asn Glu Val Tyr Pro Val Asn Tyr Asn Val Leu Ser
1               5                   10                  15

Ser Asp Ala Phe Gln Gln Leu Asp Thr Thr Gly Phe Lys Ser Lys Tyr
            20                  25                  30

Asp Glu Met Ile Lys Ala Phe Glu Lys Lys Trp Lys Lys Gly Ala Lys
        35                  40                  45

Gly Lys Asp Leu Leu Asp Val Ala Trp Thr Tyr Ile Thr Thr Gly Glu
    50                  55                  60

Ile Asp Pro Leu Asn Val Ile Lys Gly Val Leu Ser Val Leu Thr Leu
65                  70                  75                  80

Ile Pro Glu Val Gly Thr Val Ala Ser Ala Ala Ser Thr Ile Val Ser
                85                  90                  95

Phe Ile Trp Pro Lys Ile Phe Gly Asp Lys Pro Asn Ala Lys Asn Ile
            100                 105                 110

Phe Glu Glu Leu Lys Pro Gln Ile Glu Ala Leu Ile Gln Gln Asp Ile
        115                 120                 125
Thr Asn Tyr Gln Asp Ala Ile Asn Gln Lys Lys Phe Asp Ser Leu Gln
    130                 135                 140
Lys Thr Ile Asn Leu Tyr Thr Val Ala Ile Asp Asn Asn Asp Tyr Val
145                 150                 155                 160
Thr Ala Lys Thr Gln Leu Glu Asn Leu Asn Ser Ile Leu Thr Ser Asp
                165                 170                 175
Ile Ser Ile Phe Ile Pro Glu Gly Tyr Glu Thr Gly Gly Leu Pro Tyr
            180                 185                 190
Tyr Ala Met Val Ala Asn Ala His Ile Leu Leu Leu Arg Asp Ala Ile
        195                 200                 205
Val Asn Ala Glu Lys Leu Gly Phe Ser Asp Lys Glu Val Asp Thr His
    210                 215                 220
Lys Lys Tyr Ile Lys Met Thr Ile His Asn His Thr Glu Ala Val Ile
225                 230                 235                 240
Lys Ala Phe Leu Asn Gly Leu Asp Lys Phe Lys Ser Leu Asp Val Asn
                245                 250                 255
Ser Tyr Asn Lys Lys Ala Asn Tyr Ile Lys Gly Met Thr Glu Met Val
            260                 265                 270
Leu Asp Leu Val Ala Leu Trp Pro Thr Phe Asp Pro Asp His Tyr Gln
        275                 280                 285
Lys Glu Val Glu Ile Glu Phe Thr Arg Thr Ile Ser Ser Pro Ile Tyr
    290                 295                 300
Gln Pro Val Pro Lys Asn Met Gln Asn Thr Ser Ser Ser Ile Val Pro
305                 310                 315                 320
Ser Asp Leu Phe His Tyr Gln Gly Asp Leu Val Lys Leu Glu Phe Ser
                325                 330                 335
Thr Arg Thr Asp Asn Asp Gly Leu Ala Lys Ile Phe Thr Gly Ile Arg
            340                 345                 350
Asn Thr Phe Tyr Lys Ser Pro Asn Thr His Glu Thr Tyr His Val Asp
        355                 360                 365
Phe Ser Tyr Asn Thr Gln Ser Ser Gly Asn Ile Ser Arg Gly Ser Ser
    370                 375                 380
Asn Pro Ile Pro Ile Asp Leu Asn Asn Pro Ile Ile Ser Thr Cys Ile
385                 390                 395                 400
Arg Asn Ser Phe Tyr Lys Ala Ile Ala Gly Ser Ser Val Leu Val Asn
                405                 410                 415
Phe Lys Asp Gly Thr Gln Gly Tyr Ala Phe Ala Gln Ala Pro Thr Gly
            420                 425                 430
Gly Ala Trp Asp His Ser Phe Ile Glu Ser Asp Gly Ala Pro Glu Gly
        435                 440                 445
His Lys Leu Asn Tyr Ile Tyr Thr Ser Pro Gly Asp Thr Leu Arg Asp
    450                 455                 460
Phe Ile Asn Val Tyr Thr Leu Ile Ser Thr Pro Thr Ile Asn Glu Leu
465                 470                 475                 480
Ser Thr Glu Lys Ile Lys Gly Phe Pro Ala Glu Lys Gly Tyr Ile Lys
                485                 490                 495
Asn Gln Gly Ile Met Lys Tyr Tyr Gly Lys Pro Glu Tyr Ile Asn Gly
            500                 505                 510
Ala Gln Pro Val Asn Leu Glu Asn Gln Gln Thr Leu Ile Phe Glu Phe
        515                 520                 525
His Ala Ser Lys Thr Ala Gln Tyr Thr Ile Arg Ile Arg Tyr Ala Ser
    530                 535                 540
Thr Gln Gly Thr Lys Gly Tyr Phe Arg Leu Asp Asn Gln Glu Leu Gln
545                 550                 555                 560
Thr Leu Asn Ile Pro Thr Ser His Asn Gly Tyr Val Thr Gly Asn Ile
                565                 570                 575

Gly Glu Asn Tyr Asp Leu Tyr Thr Ile Gly Ser Tyr Thr Ile Thr Glu
            580                 585                 590
Gly Asn His Thr Leu Gln Ile Gln His Asn Asp Lys Asn Gly Met Val
        595                 600                 605
Leu Asp Arg Ile Glu Phe Val Pro Lys Asp Ser Leu Gln Asp Ser Pro
    610                 615                 620
Gln Asp Ser Pro Pro Glu Val His Glu Ser Thr Ile Ile Phe Asp Lys
625                 630                 635                 640
Ser Ser Pro Thr Ile Trp Ser Ser Asn Lys His Ser Tyr Ser His Ile
                645                 650                 655
His Leu Glu Gly Ser Tyr Thr Ser Gln Gly Ser Tyr Pro His Asn Leu
            660                 665                 670
Leu Ile Asn Leu Phe His Pro Thr Asp Pro Asn Arg Asn His Thr Ile
        675                 680                 685
His Val Asn Asn Gly Asp Met Asn Val Asp Tyr Gly Lys Asp Ser Val
    690                 695                 700
Ala Asp Gly Leu Asn Phe Asn Lys Ile Thr Ala Thr Ile Pro Ser Asp
705                 710                 715                 720
Ala Trp Tyr Ser Gly Thr Ile Thr Ser Met His Leu Phe Asn Asp Asn
                725                 730                 735
Asn Phe Lys Thr Ile Thr Pro Lys Phe Glu Leu Ser Asn Glu Leu Glu
            740                 745                 750
Asn Ile Thr Thr Gln Val Asn Ala Leu Phe Ala Ser Ser Ala Gln Asp
        755                 760                 765
Thr Leu Ala Ser Asn Val Ser Asp Tyr Trp Ile Glu Gln Val Val Met
    770                 775                 780
Lys Val Asp Ala Leu Ser Asp Glu Val Phe Gly Lys Glu Lys Lys Ala
785                 790                 795                 800
Leu Arg Lys Leu Val Asn Gln Ala Lys Arg Leu Ser Lys Ile Arg Asn
                805                 810                 815
Leu Leu Ile Gly Gly Asn Phe Asp Asn Leu Val Ala Trp Tyr Met Gly
            820                 825                 830
Lys Asp Val Val Lys Glu Ser Asp His Glu Leu Phe Lys Ser Asp His
        835                 840                 845
Val Leu Leu Pro Pro Pro Thr Phe His Pro Ser Tyr Ile Phe Gln Lys
    850                 855                 860
Val Glu Glu Ser Lys Leu Lys Pro Asn Thr Arg Tyr Thr Ile Ser Gly
865                 870                 875                 880
Phe Ile Ala His Gly Glu Asp Val Glu Leu Val Val Ser Arg Tyr Gly
                885                 890                 895
Gln Glu Ile Gln Lys Val Met Gln Val Pro Tyr Glu Glu Ala Leu Pro
            900                 905                 910
Leu Thr Ser Glu Ser Asn Ser Ser Cys Cys Val Pro Asn Leu Asn Ile
        915                 920                 925
Asn Glu Thr Leu Ala Asp Pro His Phe Phe Ser Tyr Ser Ile Asp Val
    930                 935                 940
Gly Ser Leu Glu Met Glu Ala Asn Pro Gly Ile Glu Phe Gly Leu Arg
945                 950                 955                 960
Ile Val Lys Pro Thr Gly Met Ala Arg Val Ser Asn Leu Glu Ile Arg
                965                 970                 975
Glu Asp Arg Pro Leu Thr Ala Lys Glu Ile Arg Gln Val Gln Arg Ala
            980                 985                 990
Ala Arg Asp Trp Lys Gln Asn Tyr Glu Gln Glu Arg Thr Glu Ile Thr
        995                 1000                1005
Ala Ile Ile Gln Pro Val Leu Asn Gln Ile Asn Ala Leu Tyr Glu Asn
    1010                1015                1020
Glu Asp Trp Asn Gly Ser Ile Arg Ser Asn Val Ser Tyr His Asp Leu
1025                1030                1035                1040

Glu Gln Ile Met Leu Pro Thr Leu Leu Lys Thr Glu Glu Ile Asn Cys
                1045                1050                1055

Asn Tyr Asp His Pro Ala Phe Leu Leu Lys Val Tyr His Trp Phe Met
            1060                1065                1070

Thr Asp Arg Ile Gly Glu His Gly Thr Ile Leu Ala Arg Phe Gln Glu
        1075                1080                1085

Ala Leu Asp Arg Ala Tyr Thr Gln Leu Glu Ser Arg Asn Leu Leu His
    1090                1095                1100

Asn Gly His Phe Thr Thr Asp Thr Ala Asn Trp Thr Ile Glu Gly Asp
1105                1110                1115                1120

Ala His His Thr Ile Leu Glu Asp Gly Arg Arg Val Leu Arg Leu Pro
                1125                1130                1135

Asp Trp Ser Ser Asn Ala Thr Gln Thr Ile Glu Ile Glu Asp Phe Asp
            1140                1145                1150

Leu Asp Gln Glu Tyr Gln Leu Leu Ile His Ala Lys Gly Lys Gly Ser
        1155                1160                1165

Ile Thr Leu Gln His Gly Glu Glu Asn Glu Tyr Val Glu Thr His Thr
    1170                1175                1180

His His Thr Asn Asp Phe Ile Thr Ser Gln Asn Ile Pro Phe Thr Phe
1185                1190                1195                1200

Lys Gly Asn Gln Ile Glu Val His Ile Thr Ser Glu Asp Gly Glu Phe
                1205                1210                1215

Leu Ile Asp His Ile Thr Val Ile Glu Val Ser Lys Thr Asp Thr Asn
            1220                1225                1230

Thr Asn Ile Ile Glu Asn Ser Pro Ile Asn Thr Ser Met Asn Ser Asn
        1235                1240                1245

Val Arg Val Asp Ile Pro Arg Ser Leu
    1250                1255

(2) INFORMATION FOR SEQ ID NO:7:

(i) SEQUENCE CHARACTERISTICS:
(A) LENGTH: 3738 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Bacillus thuringiensis
(C) INDIVIDUAL ISOLATE: PS86Q3

(vii) IMMEDIATE SOURCE:
(A) LIBRARY: Lambdagem (TM) - 11 LIBRARY
(B) CLONE: 86Q3a

(xi) SEQUENCE DESCRIPTION: SEQ ID NO:7:

ATGGCAACAA TTAATGAGTT GTATCCAGTT CCTTATAATG TGCTAGCTCA TCCAATTAAA 60

GAAGTCGATG ATCCTTATTC TTGGTCAAAT TTATTAAAGG GTATACAAGA AGGTTGGGAA 120

GAATGGGGAA AAACAGGACA AAAAAAACTT TTTGAAGACC ATCTTACGAT TGCATGGAAT 180

CTTTATAAAA CAGGAAAATT AGATTATTTC GCTTTGACAA AAGCATCAAT ATCATTGATT 240

GGATTTATTC CAGGGGCAGA AGCAGCAGTT CCCTTTATTA ATATGTTTGT AGACTTTGTT 300

TGGCCTAAAT TATTTGGTGC GAATACAGAA GGAAAAGATC AACAGTTGTT TAATGCTATC 360

ATGGATGCAG TTAATAAAAT GGTAGATAAT AAGTTCTTAA GTTATAATCT TAGTACACTT 420

AATAAAACAA TTGAAGGACT TCAAGGTAAT TTAGGCCTAT TTCAAAATGC TATACAAGTA 480

GCCATTTGTC AAGGCAGTAC ACCAGAAAGA GTAAATTTTG ATCAAAATTG TACACCATGT 540

AATCCAAATC AACCTTGTAA AGATGATTTG GATAGAGTTG CTTCACGTTT TGATACGGCT 600

AATTCTCAAT TCACACAGCA TTTACCAGAA TTTAAAAATC CTTGGTCGGA TGAAAACTCT 660

ACTCAGGAAT TTAAAAGAAC ATCTGTTGAA TTAACTTTAC CAATGTATAC AACAGTAGCT 720
ACGTTACATC TTTTATTATA TGAAGGATAT ATAGAATTTA TGACAAAATG GAATTTTCAC 780
AATGAACAAT ATTTAAATAA TTTAAAGGTA GAATTACAAC AATTGATACA CTCATATTCA 840
GAAACTGTTC GTACAAGTTT CCTTCAATTT TTACCTACCT TGAATAATCG TTCAAAATCA 900
TCCGTAAATG CTTATAACCG TTATGTCCGC AATATGACTG TTAACTGTTT AGATATTGCT 960
GCTACATGGC CTACATTTGA TACACATAAT TATCATCAAG GTGGTAAATT AGATTTAACT 1020
CGTATTATTC TTTCAGATAC AGCAGGACCA ATAGAAGAAT ATACTACTGG CGACAAAACT 1080
TCAGGACCTG AACATAGTAA CATTACACCA AATAATATTC TAGATACACC ATCTCCAACA 1140
TATCAGCACT CATTTGTATC TGTTGATTCT ATTGTATATT CTAGAAAAGA ATTACAACAA 1200
TTAGACATAG CTACTTATAG TACAAATAAT AGTAATAATT GTCACCCTTA TGGATTACGA 1260
CTTTCATATA CAGATGGAAG CAGATATGAT TATGGAGATA ATCAACCTGA TTTTACTACT 1320
TCCAATAACA ATTATTGTCA TAATAGCTAT ACTGCCCCTA TTACACTTGT GAATGCACGA 1380
CATTTATATA ATGCAAAAGG CTCTTTACAA AATGTAGAAT CTTTAGTGGT TAGTACTGTA 1440
AATGGTGGAA GTGGTTCATG CATTTGTGAT GCATGGATTA ATTATTTACG TCCTCCTCAA 1500
ACAAGTAAAA ATGAATCACG TCCTGATCAA AAAATTAATG TTTTGTATCC AATAACAGAA 1560
ACTGTAAATA AGGGGACTGG AGGAAATTTA GGAGTTATTT CTGCCTATGT TCCAATGGAA 1620
CTTGTACCAG AAAACGTTAT TGGAGATGTT AATGCTGATA CTAAATTGCC ACTTACACAA 1680
TTAAAGGGCT TTCCATTTGA AAAATATGGT TCTGAGTATA ATAATCGGGG TATCTCTCTT 1740
GTTCGCGAAT GGATAAATGG TAACAATGCA GTTAAACTTT CTAATAGTCA ATCTGTTGGC 1800
ATACAAATTA CGAATCAAAC CAAACAAAAA TATGAAATAC GTTGCCGTTA TGCGAGTAAA 1860
GGAGATAATA ATGTTTATTT TAATGTGGAT TTAAGTGAAA ATCCATTTAG AAATTCCATT 1920
TCTTTTGGAT CTACTGAAAG TTCTGTTGTA GGAGTACAAG GTGAAAATGG AAAGTATATA 1980
TTGAAATCAA TCACAACGGT AGAAATACCT GCTGGAAGTT TCTATGTTCA TATAACAAAC 2040
CAAGGTTCTT CAGATCTCTT TTTAGATCGT ATTGAGTTTG TTCCAAAAAT CCAATTCCAA 2100
TTCTGTGATA ATAATAATCT TCACTGTGAT TGTAATAACC CTGTTGACAC CGATTGTACA 2160
TTTTGTTGCG TTTGCACTAG TCTTACTGAT TGTGATTGTA ATAACCCTCG TGGCCTAGAT 2220
TGTACGCTAT GTTGTCAGGT AGAAAATCAG CTACCTTCTT TTGTGACACT TACAGATTTA 2280
CAAAATATTA CGACACAAGT AAATGCATTA GTTGCATCGA GCGAACATGA TACACTTGCA 2340
ACAGACGTGA GTGATTATGA GATTGAAGAA GTTGTACTGA AAGTAGATGC ATTATCTGGT 2400
GAAGTGTTTG GAAAAGAGAA AAAAGCATTG CGTAAATTGG TAAATCACAC AAAACGTTTA 2460
AGCAAAGCGC GTAACCTCTT GATAGGAGGA AATTTTGATA ACTTGGATGC TTGGTACAGA 2520
GGCCGAAATG TAGTAAACGT ATCTGATCAT GAACTATTTA AGAGTGATCA TGTATTATTG 2580
CCACCACCAA CACTGTACTC ATCTTATATG TTCCAAAAAG TAGAGGAATC GAAATTAAAA 2640
GCGAATACAC GTTATACTGT GTCTGGTTTT ATTGCACATG CAGAAGATTT AGAAATTGTT 2700
GTGTCTCGTT ATGGGCAAGA AGTGAAGAAA GTGGTTCAAG TTCCATATGG AGAAGCATTC 2760
CCATTGACAT CGAGGGGAGC GATTTGTTGC CCTCCACGTT CTACAAGTAA TGGAAAACCT 2820
GCTGATCCAC ATTTCTTTAG TTACAGTATT GATGTGGGAA CATTAGATGT AGAAGCAAAC 2880
CCTGGTATCG AATTGGGTCT TCGTATTGTA GAACGAACTG GAATGGCACG TGTAAGTAAT 2940
TTAGAAATTC GTGAAGATCG TCCATTAAAG AAAAATGAAC TCCGCAATGT ACAACGTGCA 3000
GCAAGAAATT GGAGAACAGC ATATGACCAA GAACGTGCAG AAGTAACGGC CTTGATTCAA 3060
CCTGTATTAA ATCAAATCAA TGCGTTGTAT GAAAATGAAG ATTGGAATGG AGCAATTCGT 3120
TCTGGAGTTT CTTATCATGA CTTAGAAGCA ATTGTTTTAC CAACATTACC AAAATTAAAT 3180
CATTGGTTTA TGTCTGATAT GTTAGGGGAA CAAGGTTCCA TTTTAGCTCA ATTTCAAGAA 3240
GCATTAGATC GTGCGTATAC GCAACTCGAA GAAAGTACAA TTCTGCATAA TGGTCATTTC 3300

ACAACAGATG CAGCAAATTG GACGATAGAA GGCGATGCAC ATCATGCGAT ATTAGAAGAT 3360

GGTAGACGCG TATTACGTCT TCCAGATTGG TCTTCTAGCG TTTCACAAAC CATTGAAATA 3420

GAAAATTTTG ATCCAGATAA AGAATATCAG TTAGTTTTCC ATGCACAAGG AGAAGGAACG 3480

GTCTCCCTTC AACATGGTGA AGAAGGAGAA TATGTGGAAA CACACCCGCA TAAGTCTGCG 3540

AATTTTACAA CTTCACACCG TCAAGGAGTC ACATTTGAAA CAAATAAAGT AACAGTTGAA 3600

ATTACCTCAG AAGATGGAGA ATTCCTAGTC GATCATATTG CTCTTGTGGA AGCTCCTCTT 3660

CCTACAGATG ACCAAAGTTC AGATGGAAAT ACGACTTCCA ATACGAATAG CAATACAAGT 3720

ATGAATAATA ATCAATAA 3738

(2) INFORMATION FOR SEQ ID NO:8:

(i) SEQUENCE CHARACTERISTICS:
(A) LENGTH: 1245 amino acids
(B) TYPE: amino acid
(C) STRANDEDNESS: single
(D) TOPOLOGY: linear

(ii) MOLECULE TYPE: protein

(iii) HYPOTHETICAL: YES

(iv) ANTI-SENSE: NO

(vi) ORIGINAL SOURCE:
(A) ORGANISM: BACILLUS THURINGIENSIS
(C) INDIVIDUAL ISOLATE: PS86Q3

(vii) IMMEDIATE SOURCE:
(A) LIBRARY: LAMBDAGEM (tm) - 11 library
(B) CLONE: 86Q3A

(xi) SEQUENCE DESCRIPTION: SEQ ID NO:8:

Met Ala Thr Ile Asn Glu Leu Tyr Pro Val Pro Tyr Asn Val Leu Ala
1               5                   10                  15

His Pro Ile Lys Glu Val Asp Asp Pro Tyr Ser Trp Ser Asn Leu Leu
            20                  25                  30

Lys Gly Ile Gln Glu Gly Trp Glu Glu Trp Gly Lys Thr Gly Gln Lys
        35                  40                  45

Lys Leu Phe Glu Asp His Leu Thr Ile Ala Trp Asn Leu Tyr Lys Thr
    50                  55                  60

Gly Lys Leu Asp Tyr Phe Ala Leu Thr Lys Ala Ser Ile Ser Leu Ile
65                  70                  75                  80

Gly Phe Ile Pro Gly Ala Glu Ala Ala Val Pro Phe Ile Asn Met Phe
                85                  90                  95

Val Asp Phe Val Trp Pro Lys Leu Phe Gly Ala Asn Thr Glu Gly Lys
            100                 105                 110

Asp Gln Gln Leu Phe Asn Ala Ile Met Asp Ala Val Asn Lys Met Val
        115                 120                 125

Asp Asn Lys Phe Leu Ser Tyr Asn Leu Ser Thr Leu Asn Lys Thr Ile
    130                 135                 140

Glu Gly Leu Gln Gly Asn Leu Gly Leu Phe Gln Asn Ala Ile Gln Val
145                 150                 155                 160

Ala Ile Cys Gln Gly Ser Thr Pro Glu Arg Val Asn Phe Asp Gln Asn
                165                 170                 175

Cys Thr Pro Cys Asn Pro Asn Gln Pro Cys Lys Asp Asp Leu Asp Arg
            180                 185                 190

Val Ala Ser Arg Phe Asp Thr Ala Asn Ser Gln Phe Thr Gln His Leu
        195                 200                 205

Pro Glu Phe Lys Asn Pro Trp Ser Asp Glu Asn Ser Thr Gln Glu Phe
    210                 215                 220

Lys Arg Thr Ser Val Glu Leu Thr Leu Pro Met Tyr Thr Thr Val Ala
225                 230                 235                 240

Thr Leu His Leu Leu Leu Tyr Glu Gly Tyr Ile Glu Phe Met Thr Lys
                245                 250                 255

Trp Asn Phe His Asn Glu Gln Tyr Leu Asn Asn Leu Lys Val Glu Leu
            260                 265                 270

Gln Gln Leu Ile His Ser Tyr Ser Glu Thr Val Arg Thr Ser Phe Leu
        275                 280                 285

Gln Phe Leu Pro Thr Leu Asn Asn Arg Ser Lys Ser Ser Val Asn Ala
    290                 295                 300

Tyr Asn Arg Tyr Val Arg Asn Met Thr Val Asn Cys Leu Asp Ile Ala
305                 310                 315                 320

Ala Thr Trp Pro Thr Phe Asp Thr His Asn Tyr His Gln Gly Gly Lys
                325                 330                 335

Leu Asp Leu Thr Arg Ile Ile Leu Ser Asp Thr Ala Gly Pro Ile Glu
            340                 345                 350

Glu Tyr Thr Thr Gly Asp Lys Thr Ser Gly Pro Glu His Ser Asn Ile
        355                 360                 365

Thr Pro Asn Asn Ile Leu Asp Thr Pro Ser Pro Thr Tyr Gln His Ser
    370                 375                 380

Phe Val Ser Val Asp Ser Ile Val Tyr Ser Arg Lys Glu Leu Gln Gln
385                 390                 395                 400

Leu Asp Ile Ala Thr Tyr Ser Thr Asn Asn Ser Asn Asn Cys His Pro
                405                 410                 415

Tyr Gly Leu Arg Leu Ser Tyr Thr Asp Gly Ser Arg Tyr Asp Tyr Gly
            420                 425                 430

Asp Asn Gln Pro Asp Phe Thr Thr Ser Asn Asn Asn Tyr Cys His Asn
        435                 440                 445

Ser Tyr Thr Ala Pro Ile Thr Leu Val Asn Ala Arg His Leu Tyr Asn
    450                 455                 460

Ala Lys Gly Ser Leu Gln Asn Val Glu Ser Leu Val Val Ser Thr Val
465                 470                 475                 480

Asn Gly Gly Ser Gly Ser Cys Ile Cys Asp Ala Trp Ile Asn Tyr Leu
                485                 490                 495

Arg Pro Pro Gln Thr Ser Lys Asn Glu Ser Arg Pro Asp Gln Lys Ile
            500                 505                 510

Asn Val Leu Tyr Pro Ile Thr Glu Thr Val Asn Lys Gly Thr Gly Gly
        515                 520                 525

Asn Leu Gly Val Ile Ser Ala Tyr Val Pro Met Glu Leu Val Pro Glu
    530                 535                 540

Asn Val Ile Gly Asp Val Asn Ala Asp Thr Lys Leu Pro Leu Thr Gln
545                 550                 555                 560

Leu Lys Gly Phe Pro Phe Glu Lys Tyr Gly Ser Glu Tyr Asn Asn Arg
                565                 570                 575

Gly Ile Ser Leu Val Arg Glu Trp Ile Asn Gly Asn Asn Ala Val Lys
            580                 585                 590

Leu Ser Asn Ser Gln Ser Val Gly Ile Gln Ile Thr Asn Gln Thr Lys
        595                 600                 605

Gln Lys Tyr Glu Ile Arg Cys Arg Tyr Ala Ser Lys Gly Asp Asn Asn
    610                 615                 620

Val Tyr Phe Asn Val Asp Leu Ser Glu Asn Pro Phe Arg Asn Ser Ile
625                 630                 635                 640

Ser Phe Gly Ser Thr Glu Ser Ser Val Val Gly Val Gln Gly Glu Asn
                645                 650                 655

Gly Lys Tyr Ile Leu Lys Ser Ile Thr Thr Val Glu Ile Pro Ala Gly
            660                 665                 670

Ser Phe Tyr Val His Ile Thr Asn Gln Gly Ser Ser Asp Leu Phe Leu
        675                 680                 685

Asp Arg Ile Glu Phe Val Pro Lys Ile Gln Phe Gln Phe Cys Asp Asn
    690                 695                 700

Asn Asn Leu His Cys Asp Cys Asn Asn Pro Val Asp Thr Asp Cys Thr
705                 710                 715                 720

Phe Cys Cys Val Cys Thr Ser Leu Thr Asp Cys Asp Cys Asn Asn Pro
                725                 730                 735

Arg Gly Leu Asp Cys Thr Leu Cys Cys Gln Val Glu Asn Gln Leu Pro
            740                 745                 750

Ser Phe Val Thr Leu Thr Asp Leu Gln Asn Ile Thr Thr Gln Val Asn
        755                 760                 765

Ala Leu Val Ala Ser Ser Glu His Asp Thr Leu Ala Thr Asp Val Ser
    770                 775                 780

Asp Tyr Glu Ile Glu Glu Val Val Leu Lys Val Asp Ala Leu Ser Gly
785                 790                 795                 800

Glu Val Phe Gly Lys Glu Lys Lys Ala Leu Arg Lys Leu Val Asn His
                805                 810                 815

Thr Lys Arg Leu Ser Lys Ala Arg Asn Leu Leu Ile Gly Gly Asn Phe
            820                 825                 830

Asp Asn Leu Asp Ala Trp Tyr Arg Gly Arg Asn Val Val Asn Val Ser
        835                 840                 845

Asp His Glu Leu Phe Lys Ser Asp His Val Leu Leu Pro Pro Pro Thr
    850                 855                 860

Leu Tyr Ser Ser Tyr Met Phe Gln Lys Val Glu Glu Ser Lys Leu Lys
865                 870                 875                 880

Ala Asn Thr Arg Tyr Thr Val Ser Gly Phe Ile Ala His Ala Glu Asp
                885                 890                 895

Leu Glu Ile Val Val Ser Arg Tyr Gly Gln Glu Val Lys Lys Val Val
            900                 905                 910

Gln Val Pro Tyr Gly Glu Ala Phe Pro Leu Thr Ser Arg Gly Ala Ile
        915                 920                 925

Cys Cys Pro Pro Arg Ser Thr Ser Asn Gly Lys Pro Ala Asp Pro His
    930                 935                 940

Phe Phe Ser Tyr Ser Ile Asp Val Gly Thr Leu Asp Val Glu Ala Asn
945                 950                 955                 960

Pro Gly Ile Glu Leu Gly Leu Arg Ile Val Glu Arg Thr Gly Met Ala
                965                 970                 975

Arg Val Ser Asn Leu Glu Ile Arg Glu Asp Arg Pro Leu Lys Lys Asn
            980                 985                 990

Glu Leu Arg Asn Val Gln Arg Ala Ala Arg Asn Trp Arg Thr Ala Tyr
        995                 1000                1005

Asp Gln Glu Arg Ala Glu Val Thr Ala Leu Ile Gln Pro Val Leu Asn
    1010                1015                1020

Gln Ile Asn Ala Leu Tyr Glu Asn Glu Asp Trp Asn Gly Ala Ile Arg
1025                1030                1035                1040

Ser Gly Val Ser Tyr His Asp Leu Glu Ala Ile Val Leu Pro Thr Leu
                1045                1050                1055

Pro Lys Leu Asn His Trp Phe Met Ser Asp Met Leu Gly Glu Gln Gly
            1060                1065                1070

Ser Ile Leu Ala Gln Phe Gln Glu Ala Leu Asp Arg Ala Tyr Thr Gln
        1075                1080                1085

Leu Glu Glu Ser Thr Ile Leu His Asn Gly His Phe Thr Thr Asp Ala
    1090                1095                1100

Ala Asn Trp Thr Ile Glu Gly Asp Ala His His Ala Ile Leu Glu Asp
1105                1110                1115                1120

Gly Arg Arg Val Leu Arg Leu Pro Asp Trp Ser Ser Ser Val Ser Gln
                1125                1130                1135

Thr Ile Glu Ile Glu Asn Phe Asp Pro Asp Lys Glu Tyr Gln Leu Val
            1140                1145                1150

Phe His Ala Gln Gly Glu Gly Thr Val Ser Leu Gln His Gly Glu Glu
        1155                1160                1165

Gly Glu Tyr Val Glu Thr His Pro His Lys Ser Ala Asn Phe Thr Thr
    1170                1175                1180

Ser His Arg Gln Gly Val Thr Phe Glu Thr Asn Lys Val Thr Val Glu
1185                1190                1195                1200

Ile Thr Ser Glu Asp Gly Glu Phe Leu Val Asp His Ile Ala Leu Val
                1205                1210                1215

Glu Ala Pro Leu Pro Thr Asp Asp Gln Ser Ser Asp Gly Asn Thr Thr
            1220                1225                1230

Ser Asn Thr Asn Ser Asn Thr Ser Met Asn Asn Asn Gln
        1235                1240                1245

(2) INFORMATION FOR SEQ ID NO:9:

(i) SEQUENCE CHARACTERISTICS:
(A) LENGTH: 2412 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(vi) ORIGINAL SOURCE:
(A) ORGANISM: Bacillus thuringiensis
(C) INDIVIDUAL ISOLATE: PS63B

(vii) IMMEDIATE SOURCE:
(B) CLONE: E. coli NM522(pMYC1642) NRRL B-18961

(xi) SEQUENCE DESCRIPTION: SEQ ID NO:9:

ATGACTTGTC AATTACAAGC GCAACCACTT ATTCCCTATA ACGTACTAGC AGGAGTTCCA      60
ACTAGTAATA CAGGTAGTCC AATCGGCAAT GCAGGTAATC AATTTGATCA GTTTGAGCAA     120
ACCGTTAAAG AGCTCAAGGA AGCATGGGAA GCGTTCCAAA AAAACGGAAG TTTCTCATTA     180
GCAGCTCTTG AAAAGGGATT TGATGCAGCA ATCGGAGGAG GATCCTTTGA TTATTTAGGT     240
TTAGTTCAAG CCGGCCTAGG ATTAGTTGGT ACGCTAGGCG CCGCAATCCC TGGTGTTTCA     300
GTGGCAGTGC CTCTTATTAG CATGCTTGTT GGTGTTTTTT GGCCAAAGGG CACAAACAAC     360
CAAGAAAACC TTATTACAGT TATTGATAAG GAAGTTCAGA GAATACTAGA TGAAAAGCTA     420
TCTGATCAGT TAATAAAGAA ATTGAACGCA GATTTAAATG CTTTTACGGA CCTAGTAACT     480
CGTTTGGAAG AAGTAATAAT AGATGCAACT TTCGAGAATC ACAAGCCTGT ACTACAAGTA     540
AGTAAATCAA ATTATATGAA AGTGGATTCA GCATATTTCT CAACAGGAGG TATTCTTACT     600
CTTGGCATGA GTGATTTTCT TACTGATACC TATTCAAAGC TTACCTTCCC ATTATATGTA     660
CTAGGCGCAA CTATGAAACT TTCAGCATAT CATAGTTATA TACAATTCGG AAATACATGG     720
CTTAATAAAG TTTATGATTT ATCATCAGAT GAGGGAAAAA CAATGTCGCA GGCTTTAGCA     780
CGAGCTAAAC AGCATATGCG CCAAGACATA GCATTTTATA CAAGCCAAGC TTTAAACATG     840
TTTACTGGGA ATCTCCCTTC ATTATCATCT AATAAATATG CAATTAATGA CTATAATGTA     900
TACACTCGAG CAATGGTATT GAATGGCTTA GATATAGTAG CAACATGGCC TACCCTATAT     960
CCAGATGACT ATTCGTCTCA GATAAAACTG GAGAAAACAC GCGTGATCTT TTCAGATATG    1020
GTCGGGCAAA GTGAGAGTAG AGATGGCAGC GTAACGATTA AAAATATTTT TGACAATACA    1080
GATTCACATC AACATGGATC CATAGGTCTC AATTCAATCT CTTATTTCCC AGATGAGTTA    1140
CAGAAAGCAC AACTTCGCAT GTATGATTAT AATCACAAAC CTTATTGTAC GGACTGTTTC    1200
TGCTGGCCGT ATGGAGTGAT TTTAAACTAT AACAAGAATA CCTTTAGATA TGGCGATAAT    1260
GATCCAGGTC TTTCAGGAGA CGTTCAACTC CCAGCACCTA TGAGTGTAGT TAATGCCCAA    1320
ACTCAAACAG CCCAATATAC AGATGGAGAA AACATATGGA CAGATACTGG CCGCAGTTGG    1380
CTTTGTACTC TACGTGGCTA CTGTACTACA AACTGTTTTC CAGGAAGAGG TTGTTATAAT    1440
AATAGTACTG GATATGGAGA AAGTTGCAAT CAATCACTTC CAGGTCAAAA AATACATGCA    1500
CTATATCCTT TTACACAAAC AAATGTGCTG GGACAATCAG GCAAACTAGG ATTGCTAGCA    1560
AGTCATATTC CATATGACCT AAGTCCGAAC AATACGATTG GTGACAAAGA TACAGATTCT    1620

ACGAATATTG TCGCAAAAGG AATTCCAGTG GAAAAAGGGT ATGCATCCAG TGGACAAAAA 1680
GTTGAAATTA TACGAGAGTG GATAAATGGT GCGAATGTAG TTCAATTATC TCCAGGCCAA 1740
TCTTGGGGAA TGGATTTTAC CAATAGCACA GGTGGTCAAT ATATGGTCCG CTGTCGATAT 1800
GCAAGTACAA ACGATACTCC AATCTTTTTT AATTTAGTGT ATGACGGGGG ATCGAATCCT 1860
ATTTATAACC AGATGACATT CCCTGCTACA AAAGAGACTC CAGCTCACGA TTCAGTAGAT 1920
AACAAGATAC TAGGCATAAA AGGAATAAAT GGAAATTATT CACTCATGAA TGTAAAAGAT 1980
TCTGTCGAAC TTCCATCTGG GAAATTTCAT GTTTTTTTCA CAAATAATGG ATCATCTGCT 2040
ATTTATTTAG ATCGACTTGA GTTTGTTCCT TTAGATCAAC CAGCAGCGCC AACACAGTCA 2100
ACACAACCAA TTAATTATCC TATCACAAGT AGGTTACCTC ATCGTTCCGG AGAACCACCT 2160
GCAATAATAT GGGAGAAATC AGGGAATGTT CGCGGGAATC AACTAACTAT ATCGGCACAA 2220
GGTGTTCCAG AAAATTCCCA AATATATCTT TCGGTGGGTG GCGATCGCCA AATTTTAGAC 2280
CGTAGCAACG GATTTAAATT AGTTAATTAC TCACCTACTT ATTCTTTCAC TAACATTCAG 2340
GCTAGCTCGT CAAATTTAGT AGATATTACA AGTGGTACCA TCACTGGCCA AGTACAAGTA 2400
TCTAATCTAT AA 2412

(2) INFORMATION FOR SEQ ID NO:10:

(i) SEQUENCE CHARACTERISTICS:
(A) LENGTH: 803 amino acids
(B) TYPE: amino acid
(C) STRANDEDNESS: single
(D) TOPOLOGY: linear

(ii) MOLECULE TYPE: protein

(iii) HYPOTHETICAL: YES

(iv) ANTI-SENSE: NO

(vi) ORIGINAL SOURCE:
(A) ORGANISM: Bacillus thuringiensis
(C) INDIVIDUAL ISOLATE: PS63B

(vii) IMMEDIATE SOURCE:
(B) CLONE: E. coli NM522(pMYC1642) NRRL B-18961

(xi) SEQUENCE DESCRIPTION: SEQ ID NO:10:

Met Thr Cys Gln Leu Gln Ala Gln Pro Leu Ile Pro Tyr Asn Val Leu
1               5                   10                  15

Ala Gly Val Pro Thr Ser Asn Thr Gly Ser Pro Ile Gly Asn Ala Gly
            20                  25                  30

Asn Gln Phe Asp Gln Phe Glu Gln Thr Val Lys Glu Leu Lys Glu Ala
        35                  40                  45

Trp Glu Ala Phe Gln Lys Asn Gly Ser Phe Ser Leu Ala Ala Leu Glu
    50                  55                  60

Lys Gly Phe Asp Ala Ala Ile Gly Gly Gly Ser Phe Asp Tyr Leu Gly
65                  70                  75                  80

Leu Val Gln Ala Gly Leu Gly Leu Val Gly Thr Leu Gly Ala Ala Ile
                85                  90                  95

Pro Gly Val Ser Val Ala Val Pro Leu Ile Ser Met Leu Val Gly Val
            100                 105                 110

Phe Trp Pro Lys Gly Thr Asn Asn Gln Glu Asn Leu Ile Thr Val Ile
        115                 120                 125

Asp Lys Glu Val Gln Arg Ile Leu Asp Glu Lys Leu Ser Asp Gln Leu
    130                 135                 140

Ile Lys Lys Leu Asn Ala Asp Leu Asn Ala Phe Thr Asp Leu Val Thr
145                 150                 155                 160

Arg Leu Glu Glu Val Ile Ile Asp Ala Thr Phe Glu Asn His Lys Pro
                165                 170                 175

Val Leu Gln Val Ser Lys Ser Asn Tyr Met Lys Val Asp Ser Ala Tyr
            180                 185                 190

Phe Ser Thr Gly Gly Ile Leu Thr Leu Gly Met Ser Asp Phe Leu Thr
        195                 200                 205

Asp Thr Tyr Ser Lys Leu Thr Phe Pro Leu Tyr Val Leu Gly Ala Thr
    210                 215                 220

Met Lys Leu Ser Ala Tyr His Ser Tyr Ile Gln Phe Gly Asn Thr Trp
225                 230                 235                 240

Leu Asn Lys Val Tyr Asp Leu Ser Ser Asp Glu Gly Lys Thr Met Ser
                245                 250                 255

Gln Ala Leu Ala Arg Ala Lys Gln His Met Arg Gln Asp Ile Ala Phe
            260                 265                 270

Tyr Thr Ser Gln Ala Leu Asn Met Phe Thr Gly Asn Leu Pro Ser Leu
        275                 280                 285

Ser Ser Asn Lys Tyr Ala Ile Asn Asp Tyr Asn Val Tyr Thr Arg Ala
    290                 295                 300

Met Val Leu Asn Gly Leu Asp Ile Val Ala Thr Trp Pro Thr Leu Tyr
305                 310                 315                 320

Pro Asp Asp Tyr Ser Ser Gln Ile Lys Leu Glu Lys Thr Arg Val Ile
                325                 330                 335

Phe Ser Asp Met Val Gly Gln Ser Glu Ser Arg Asp Gly Ser Val Thr
            340                 345                 350

Ile Lys Asn Ile Phe Asp Asn Thr Asp Ser His Gln His Gly Ser Ile
        355                 360                 365

Gly Leu Asn Ser Ile Ser Tyr Phe Pro Asp Glu Leu Gln Lys Ala Gln
    370                 375                 380

Leu Arg Met Tyr Asp Tyr Asn His Lys Pro Tyr Cys Thr Asp Cys Phe
385                 390                 395                 400

Cys Trp Pro Tyr Gly Val Ile Leu Asn Tyr Asn Lys Asn Thr Phe Arg
                405                 410                 415

Tyr Gly Asp Asn Asp Pro Gly Leu Ser Gly Asp Val Gln Leu Pro Ala
            420                 425                 430

Pro Met Ser Val Val Asn Ala Gln Thr Gln Thr Ala Gln Tyr Thr Asp
        435                 440                 445

Gly Glu Asn Ile Trp Thr Asp Thr Gly Arg Ser Trp Leu Cys Thr Leu
    450                 455                 460

Arg Gly Tyr Cys Thr Thr Asn Cys Phe Pro Gly Arg Gly Cys Tyr Asn
465                 470                 475                 480

Asn Ser Thr Gly Tyr Gly Glu Ser Cys Asn Gln Ser Leu Pro Gly Gln
                485                 490                 495

Lys Ile His Ala Leu Tyr Pro Phe Thr Gln Thr Asn Val Leu Gly Gln
            500                 505                 510

Ser Gly Lys Leu Gly Leu Leu Ala Ser His Ile Pro Tyr Asp Leu Ser
        515                 520                 525

Pro Asn Asn Thr Ile Gly Asp Lys Asp Thr Asp Ser Thr Asn Ile Val
    530                 535                 540

Ala Lys Gly Ile Pro Val Glu Lys Gly Tyr Ala Ser Ser Gly Gln Lys
545                 550                 555                 560

Val Glu Ile Ile Arg Glu Trp Ile Asn Gly Ala Asn Val Val Gln Leu
                565                 570                 575

Ser Pro Gly Gln Ser Trp Gly Met Asp Phe Thr Asn Ser Thr Gly Gly
            580                 585                 590

Gln Tyr Met Val Arg Cys Arg Tyr Ala Ser Thr Asn Asp Thr Pro Ile
        595                 600                 605

Phe Phe Asn Leu Val Tyr Asp Gly Gly Ser Asn Pro Ile Tyr Asn Gln
    610                 615                 620

Met Thr Phe Pro Ala Thr Lys Glu Thr Pro Ala His Asp Ser Val Asp
625                 630                 635                 640

Asn Lys Ile Leu Gly Ile Lys Gly Ile Asn Gly Asn Tyr Ser Leu Met
                645                 650                 655

Asn Val Lys Asp Ser Val Glu Leu Pro Ser Gly Lys Phe His Val Phe
            660                 665                 670

Phe Thr Asn Asn Gly Ser Ser Ala Ile Tyr Leu Asp Arg Leu Glu Phe
        675                 680                 685

Val Pro Leu Asp Gln Pro Ala Ala Pro Thr Gln Ser Thr Gln Pro Ile
    690                 695                 700

Asn Tyr Pro Ile Thr Ser Arg Leu Pro His Arg Ser Gly Glu Pro Pro
705                 710                 715                 720

Ala Ile Ile Trp Glu Lys Ser Gly Asn Val Arg Gly Asn Gln Leu Thr
                725                 730                 735

Ile Ser Ala Gln Gly Val Pro Glu Asn Ser Gln Ile Tyr Leu Ser Val
            740                 745                 750

Gly Gly Asp Arg Gln Ile Leu Asp Arg Ser Asn Gly Phe Lys Leu Val
        755                 760                 765

Asn Tyr Ser Pro Thr Tyr Ser Phe Thr Asn Ile Gln Ala Ser Ser Ser
    770                 775                 780

Asn Leu Val Asp Ile Thr Ser Gly Thr Ile Thr Gly Gln Val Gln Val
785                 790                 795                 800

Ser Asn Leu

(2) INFORMATION FOR SEQ ID NO:11:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:11:

Arg Glu Trp Ile Asn Gly Ala Asn
1               5

(2) INFORMATION FOR SEQ ID NO:12:

(i) SEQUENCE CHARACTERISTICS:
(A) LENGTH: 21 bases
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:12:

AGARTRKWTW AATGGWGCKM A 21

(2) INFORMATION FOR SEQ ID NO:13:

(i) SEQUENCE CHARACTERISTICS:
(A) LENGTH: 20 bases
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:13:

GARTGGWTAA ATGGTRMSAA 20

(2) INFORMATION FOR SEQ ID NO:14:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:14:

Pro Thr Phe Asp Pro Asp Leu Tyr
1               5

(2) INFORMATION FOR SEQ ID NO:15:

(i) SEQUENCE CHARACTERISTICS:
(A) LENGTH: 24 bases
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:15:

CCNACYTTTK ATCCAGATSW YTAT                                                24

(2) INFORMATION FOR SEQ ID NO:16:

(i) SEQUENCE CHARACTERISTICS:
(A) LENGTH: 24 bases
(B) TYPE: nucleic acid
(C) STRANDEDNESS: single
(D) TOPOLOGY: linear

(ii) MOLECULE TYPE: DNA (synthetic)

(xi) SEQUENCE DESCRIPTION: SEQ ID NO:16:

CCWACWTTYG ATMCASATMW TTAT                                                24

(2) INFORMATION FOR SEQ ID NO:17:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:17:

Ala Ile Leu Asn Glu Leu Tyr Pro Ser Val Pro Tyr Asn Val
1               5                   10

(2) INFORMATION FOR SEQ ID NO:18:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:18:

Ala Ile Leu Asn Glu Leu Tyr Pro Ser Val Pro Tyr Asn Val
1               5                   10

(2) INFORMATION FOR SEQ ID NO:19:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:19:

Met Ala Thr Ile Asn Glu Leu Tyr Pro Asn Val Pro Tyr Asn Val Leu
1               5                   10                  15

(2) INFORMATION FOR SEQ ID NO:20:

(i) SEQUENCE CHARACTERISTICS:
(A) LENGTH: 14 amino acids
(B) TYPE: amino acid
(C) STRANDEDNESS: single

(D) TOPOLOGY: linear
(ii) MOLECULE TYPE: protein
(xi) SEQUENCE DESCRIPTION: SEQ ID NO:20:
Gln Leu Gln Ala Gln Pro Leu Ile Pro Tyr Asn Val Leu Ala
1               5                   10

(2) INFORMATION FOR SEQ ID NO:21:
(i) SEQUENCE CHARACTERISTICS:
(A) LENGTH: 10 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:21:
Ala Thr Leu Asn Glu Val Tyr Pro Val Asn
1               5                   10

(2) INFORMATION FOR SEQ ID NO:22:
(i) SEQUENCE CHARACTERISTICS:
(A) LENGTH: 15 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:22:
Val Gln Arg Ile Leu Asp Glu Lys Leu Ser Phe Gln Leu Ile Lys
1               5                   10                  15

(2) INFORMATION FOR SEQ ID NO:23:
(i) SEQUENCE CHARACTERISTICS:
(A) LENGTH: 23 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:23:
GCAATTTTAA ATGAATTATA TCC 23

(2) INFORMATION FOR SEQ ID NO:24:
(i) SEQUENCE CHARACTERISTICS:
(A) LENGTH: 17 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:24:
CAAYTACAAG CWCAACC 17

(2) INFORMATION FOR SEQ ID NO:25:
(i) SEQUENCE CHARACTERISTICS:
(A) LENGTH: 21 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:25:
TTCATCTAAA ATTCTTTGWA C 21

(2) INFORMATION FOR SEQ ID NO:26:
(i) SEQUENCE CHARACTERISTICS:
(A) LENGTH: 21 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:26:
GCWACWTTAA ATGAAGTWTA T 21

(2) INFORMATION FOR SEQ ID NO:27:
(i) SEQUENCE CHARACTERISTICS:
(A) LENGTH: 21 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:27:
AATGAAGTWT ATCCWGTWAA T 21

(2) INFORMATION FOR SEQ ID NO:28:
(i) SEQUENCE CHARACTERISTICS:
(A) LENGTH: 38 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:28:
GCAAGCGGCC GCTTATGGAA TAAATTCAAT TYKRTCWA 38

(2) INFORMATION FOR SEQ ID NO:29:
(i) SEQUENCE CHARACTERISTICS:
(A) LENGTH: 37 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:29:
AGACTGGATC CATGGCWACW ATWAATGAAT TATAYCC 37

(2) INFORMATION FOR SEQ ID NO:30:
(i) SEQUENCE CHARACTERISTICS:
(A) LENGTH: 10 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:30:
Glu Ser Lys Leu Lys Pro Asn Thr Arg Tyr
1               5                   10

(2) INFORMATION FOR SEQ ID NO:31:
(i) SEQUENCE CHARACTERISTICS:
(A) LENGTH: 29 bases
(B) TYPE: nucleic acid
(C) STRANDEDNESS: single
(D) TOPOLOGY: linear
(ii) MOLECULE TYPE: DNA (synthetic)

(xi) SEQUENCE DESCRIPTION: SEQ ID NO:31:
TAACGTGTAT WCGSTTTTAA TTTWGAYTC 29

(2) INFORMATION FOR SEQ ID NO:32:
(i) SEQUENCE CHARACTERISTICS:
(A) LENGTH: 9 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:32:
Tyr Ile Asp Lys Ile Glu Phe Ile Pro
1               5

(2) INFORMATION FOR SEQ ID NO:33:
(i) SEQUENCE CHARACTERISTICS:
(A) LENGTH: 23 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:33:
TGGAATAAAT TCAATTYKRT CWA 23

(2) INFORMATION FOR SEQ ID NO:34:
(i) SEQUENCE CHARACTERISTICS:
(A) LENGTH: 23 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:34:
AGGAACAAAY TCAAKWCGRT CTA 23

(2) INFORMATION FOR SEQ ID NO:35:
(xi) SEQUENCE DESCRIPTION: SEQ ID NO:35:
TTTAGATCGT MTTGARTTTR TWCC 24

(2) INFORMATION FOR SEQ ID NO:36:
(i) SEQUENCE CHARACTERISTICS:
(A) LENGTH: 5 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:36:
Ile Thr Ser Glu Asp
1               5

(2) INFORMATION FOR SEQ ID NO:37:
(i) SEQUENCE CHARACTERISTICS:
(A) LENGTH: 20 bases
(B) TYPE: nucleic acid
(C) STRANDEDNESS: single
(D) TOPOLOGY: linear
(ii) MOLECULE TYPE: DNA (synthetic)
(xi) SEQUENCE DESCRIPTION: SEQ ID NO:37:
TCTCCATCTT CTGARGWAAT 20

(2) INFORMATION FOR SEQ ID NO:38:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:38:

Leu Asp Arg Ile Glu Phe Val Pro
1               5